# EUROPÄISCHE PATENTSCHRIFT

(11) **EP 2 691 501 B1**
(45) Veröffentlichungstag und Bekanntmachung des Hinweises auf die Patenterteilung: **23.11.2016**
(21) Anmeldenummer: 12710507.0
(22) Anmeldetag: 23.03.2012
(51) Int. Cl.: C11B 9/00, A23L 27/20, C11D 3/50

(54) **RIECHSTOFFZUSAMMENSETZUNG MIT ALLYLETHERN ALS RIECHSTOFFVORLÄUFER**
ODORANT COMPOSITION CONTAINING ALLYL ETHERS AS ODORANT PRECURSORS
COMPOSITION DE SUBSTANCE ODORANTE CONTENANT DES ÉTHERS ALLYLIQUES EN TANT QUE PRÉCURSEUR DE SUBSTANCE ODORANTE

(30) Priorität: 29.03.2011 DE 102011006314
(43) Veröffentlichungstag der Anmeldung: 05.02.2014
(73) Patentinhaber: Henkel AG & Co. KGaA, 40589 Düsseldorf (DE)
(72) Erfinder: KROPF, Christian, 40724 Hilden (DE); GERKE, Thomas, 40627 Düsseldorf (DE); HUCHEL, Ursula, 50733 Köln (DE); MÜLLER, Thomas J.J., 40591 Düsseldorf (DE); NORDMANN, Jan, 41539 Dormagen (DE)
(86) Internationale Anmeldenummer: PCT/EP2012/055158
(87) Internationale Veröffentlichungsnummer: WO 2012/130739

(56) Entgegenhaltungen:
- EP-A1- 0 116 127
- US-A- 4 150 000
- US-A- 4 902 672
- US-B1- 6 340 666

## Beschreibung

Die vorliegende Erfindung liegt auf dem Gebiet der Riechstoffvorläufer, welche die Freisetzung von Riechstoffen ermöglichen und betrifft insbesondere Vorläuferverbindungen auf Allyletherbasis. Weiterhin betrifft die Erfindung den Einsatz entsprechender Riechstoffvorläufer in Riechstoffzusammensetzungen sowie in parfümierten oder aromatisierten Verbrauchsprodukten, wie z.B. Wasch- oder Reinigungsmitteln. Sie betrifft weiterhin Textilbeduftungsverfahren sowie Aromagebungsverfahren bei der Nahrungszubereitung.

Das Funktionsprinzip von Riechstoffvorläuferverbindungen ist grundsätzlich bekannt. Es beruht in der Regel darauf, Riechstoffe in Verbindungen umzuwandeln, welche keinen direkten Duft- oder Aromaeindruck hervorrufen, welche aber auf einen bestimmten Reiz hin, z.B. bei Einwirkung von Hitze oder Säure, den ursprünglichen Riechstoff freizusetzen vermögen und somit duft- bzw. aromawirksam werden. Auf diese Weise soll der Riechstoff bis zur gezielten Freisetzung geschützt werden.

Bekannte Riechstoffvorläufer sind z.B. die Kieselsäureester. Solche werden in der Deutschen Offenlegungsschrift DE198 41 147 A1 beschrieben. Diese Kieselsäureester enthalten die Reste von Duftstoffalkoholen, wie z.B. Octan-1-ol, und eignen sich zur Beduftung von Wasch- und Reinigungsmitteln, da sie bei der Hydrolyse die duftenden Alkohole freisetzen.

Weiterhin sind Riechstoffvorläufer auf Oxazolidinbasis bekannt. Solche werden in der Deutschen Offenlegungsschrift DE 10 2006 003 092 A1 beschrieben. Es handelt sich bei den dort beschriebenen Riechstoffvorläufern um bicyclische Oxazolidin-Derivate von Duftketonen oder-aldehyden, wie z.B. Decanal, welche bei der Hydrolyse die duftenden Aldehyde oder Ketone freizusetzen vermögen.

Weiterhin sind photolabile Riechstoffvorläufer bekannt, welche die photoindizierte Freisetzung von Riechstoffen ermöglichen. Solche werden in der Deutschen Offenlegungsschrift DE 10 2008 016 327 A1 beschrieben. Es handelt sich bei den dort beschriebenen Systemen um bestimmte Ketone, welche die Freisetzung von duftenden Terpenoiden oder Terpenen, wie z.B. β-Phellandren, nach Einwirkung elektromagnetischer Strahlung umfassend die Wellenlängen von 200 bis 400 nm ermöglichen.

In J. Org. Chem. 1999, 64, 54-59, J. Wennerberg et al., werden Katalysatoren für die mit der Retro-En -Reaktion verwandten Umlagerung von Benzylallylethern beschrieben. In J. Am. Chem. Soc., 1973, 95, 5234-5242, H. Kwart et al., wird die thermisch induzierte Reaktion von Allylethern beschrieben. Es werden dort aber weder Riechstoffzusammensetzungen noch Wasch- oder Reinigungsmittel beschrieben.

In der US 6,340,666 B1 wird 3-Hexenyl-2-methylallyether (Z) als Duftstoff beschrieben.Die Verwendung als Duftstoffvorläufer wird nicht beschrieben. Vor diesem Hintergrund war es die Aufgabe der vorliegenden Erfindung, weitere Zusammensetzungen bereitzustellen, welche eine gezielte Riechstofffreisetzung ermöglichen.

Diese Aufgabe wird vom Gegenstand der Erfindung gelöst, nämlich einem Wasch- und Reinigungsmittel enthaltend eine Riechstoffzusammensetzung, enthaltend einen Riechstoffvorläufer, bei welchem es sich um einen Allylether der Formel (I) handelt,

R¹R²C=CR³-CR⁴R⁵-O-CHR⁶R⁷ (I),

wobei die Reste R¹, R², R³, R⁴, R⁵ ,R⁶ und R⁷, unabhängig voneinander für H oder einen Kohlenwasserstoff-Rest stehen, der acyclisch oder cyclisch, substituiert oder unsubstituiert, verzweigt oder unverzweigt sowie gesättigt oder ungesättigt sein kann, und wobei einzelne Reste R¹, R², R³, R⁴, R⁵, R⁶, R⁷ miteinander ein Ringsystem bilden können,
mit der Maßgabe, dass R¹, R², R³, R⁴ und R⁵ für Reste stehen, die in einer Verbindung der allgemeinen Formel (II)

R¹R²CH-CR³=CR⁴R⁵ (II)

einen Riechstoff ergeben, wobei vorzugsweise der Rest R³ mit einem der Reste R¹ oder R⁵ ein Ringsystem bilden kann,
und/oder mit der Maßgabe, dass R⁶ und R⁷ für Reste stehen, die in einer Verbindung der allgemeinen Formel (III)

R⁶R⁷C=O (III)

einen Riechstoff ergeben, wobei vorzugsweise die Reste R⁶ und R⁷ ein Ringsystem bilden können. Die jeweiligen Kohlenwasserstoffreste können vorteilhafterweise jeweils 1 bis vorzugsweise 15 Kohlenstoffatome umfassen.

Erfindungsgemäß ist es möglich, dass einzelne Reste R¹, R², R³, R⁴, R⁵,R⁶, R⁷ miteinander ein Ringsystem bilden. Dabei kann es sich sowohl um monocyclische als auch um bicyclische Ringsysteme handeln. Z.B. kann der Rest R³ sowohl mit dem Rest R¹ als auch mit dem Rest R⁵ ein Ringsystem, insbesondere ein bicyclisches Ringsystem bilden. Beispiele für solche Riechstoffvorläufer mit Ringbildung zwischen den Resten, welche im Sinne dieser Erfindung der Formel (I) genügen sind z.B. die Myrtenolether, wie z.B. 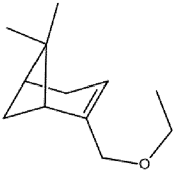 oder z.B. die Pinocarveolether, wie z.B. 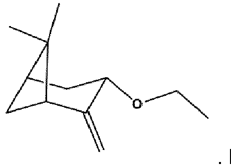 Hier liegt jeweils ein bicyclisches Ringsystem vor. Die Myrtenolether, Pinocarveolether sowie die para-Mentha-1,4(8)-dien-9-ol-ether sind besonders bevorzugte Riechstoffvorläufer im Sinne der Erfindung.

Auch ist es möglich, dass der Rest R⁶ mit dem Rest R⁷ ein Ringsystem bildet. Beispiele hierfür sind die beiden folgenden Verbindungen: 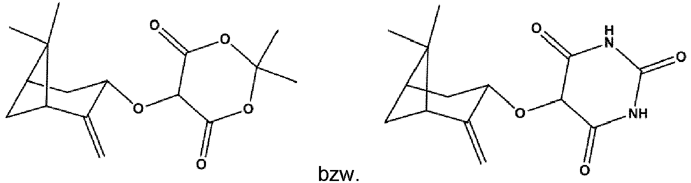 Hierwird durch die Reste Rest R⁶ und R⁷ jeweils ein monocyclisches Ringsystem gebildet.

Weitere beispielhafte Riechstoffvorläufer im Sinne der Erfindung sind die folgenden Verbindungen:

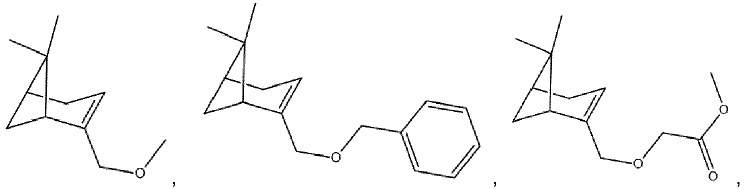

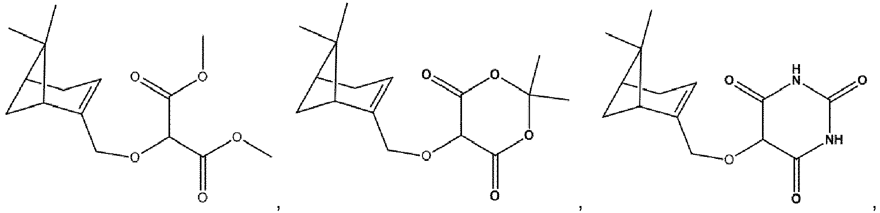

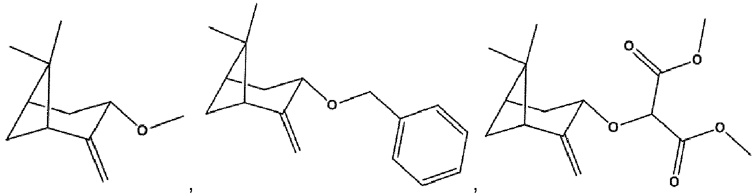

Die erfindungsgemäß eingesetzten Riechstoffvorläufer können grundsätzlich nach der Williamson-Ethersynthese dargestellt werden. Beispielsweise ist der p-Methoxybenzylallylether erhältlich durch die entsprechende Umsetzung von Anisalkohol mit Allylbromid unter Einsatz von Natriumhydrid als Base in THF. Der Octylallylether ist durch die entsprechende Umsetzung von 1-Octanol mit Allybromid unter Einsatz von Natriumhydrid als Base in THF. Der Myrtenolmethylether ist durch die entsprechende Umsetzung von Myrtenol mit lodmethan und Natriumhydrid als Base in THF erhältlich. Im Beispielteil ist weiterhin die Darstellung von trans-Pinocarveyloxy-essigsäuremethylester beschrieben.

"Riechstoffzusammensetzung" ist die Bezeichnung für eine Zusammensetzung, die zumindest einen Riechstoftvorläufer, in der Regel aber eine Vielzahl von weiteren Riechstoffen (z.B. mehr als 5, mehr als 10 oder mehr als 20 verschiedene Riechstoffe) umfasst. Die erfindungsgemäße Riechstoffzusammensetzung enthält also mindestens eine Riechstoffvorläuferverbindung gemäß Formel (I). Die erfindungsgemäße Riechstoffzusammensetzung kann neben der Riechstoffvorläuferverbindung gemäß Formel (I) einen oder mehrere weitere Riechstoffe enthalten.

"Riechstoffe" ist die Bezeichnung für chemische Verbindungen mit Geruch, welche beim Menschen ein vorzugsweise angenehmes Geruchsempfinden auslösen (Duftstoffe) und daher üblicherweise zur Parfümierung bzw. Beduftung von technischen und Sanitärartikeln, Seifen, Körperpflegemitteln, Waschmitteln, Reinigungsmitteln und dergleichen Verwendung finden. Die Begriffe Riechstoffe und Duftstoffe sind synonym zu verstehen. Von dem Begriff der Riechstoffe sind im Sinne dieser Erfindung die sogenannten Aromastoffe mitumfasst. Als Aromastoffe werden geruchsaktive Stoffe in Lebensmitteln bezeichnet. Die Wahrnehmung der sogenannten Aromastoffe in Lebensmitteln erfolgt durch Einziehen durch die Nase, also orthonasal, als auch über den Rachenraum nach oder während des Kauens bzw. Trinkens, also retronasal. Aromastoffe werden in der Regel Lebensmittel zugefügt, um diesen einen besonderen Geruch oder Geschmack zu verleihen oder einen bestehenden Geruch oder Geschmack zu veredeln oder zu modifizieren, z.B. zu verstärken. Aromastoffe sind somit zusammen mit den nicht flüchtigen Geschmacksstoffen (d.h. sauer, süß, bitter, salzig oder scharf schmeckenden Verbindungen) maßgeblich am Aroma eines Lebensmittels beteiligt. Beispielsweise ist Ethylvanillin ein bedeutender Aromastoff, da er ein viel intensiveres vanilleähnliches Aroma als Vanillin aufweist. Das Ethylvanillin wird daher regelmäßig in Lebensmitteln, Getränken und auch Futtermitteln als Aromastoff eingesetzt. Ebenso erfolgreich wird Ethylvanillin aber auch in üblichen Parfüm- und Kosmetikkompositionen als gewöhnlicher Riechstoff (Duftstoff) eingesetzt. Bevorzugte Riechstoffe werden im Verlaufe der Beschreibung noch genannt. Da von dem Begriff der Riechstoffe im Sinne dieser Erfindung auch die sogenannten Aromastoffe mitumfasst sind, gilt dies im analogen Sinne auch für den Begriff der "Aromastoffzusammensetzung" mit Bezug auf die "Riechstoftzusammensetzung". Im Sinne der Erfindung umfasst der Begriff der "Riechstoffzusammensetzung" also den Begriff der "Aromastoffzusammensetzung" sowie des Parfüms. Eine "Aromastoftzusammensetzung" im Sinne der Erfindung ist eine spezielle Riechstoffzusammensetzung, welche für den Einsatz in Lebensmitteln und damit für den Verzehr geeignet ist. Unter Parfüms versteht man alkoholische Lösungen geeigneter Riechstoffe (Duftstoffe). Eine erfindungsgemäße "Riechstoffzusammensetzung" kann selbstverständlich über die enthaltenen Riechstoffe und den erfindungsgemäßen Riechstoffvorläufer hinaus weitere typische Inhaltsstoffe, wie z.B. Lösungsmittel oder ähnliches enthalten.

Die erfindungsgemäß eingesetzten Vorläuferverbindungen der allgemeinen Formel (I) ermöglichen die gezielte Freisetzung von duftenden Aldehyden, -ketonen und/oder Riechstoffen mit allylischem Wasserstoffatom. Dabei kann die Freisetzung insbesondere thermisch und/oder säurekatalysiert induziert werden. Dabei zerfällt die Vorläuferverbindung der Formel (I) in Verbindungen der allgemeinen Formel (II) sowie der allgemeinen Formel (III) im Sinne der sogenannten Retro-En-Reaktion. Die Retro-En-Reaktion ist an sich bekannt. Es handelt sich um die Umkehrung der En-Reaktion. Unter der En-Reaktion versteht man die von Kurt Alder untersuchte Addition von Alkenen mit allylischen Wasserstoff-Atomen an sogenannte Enophile, d.h. H-Akzeptoren. Dabei sind die Enophile nicht auf die Gruppe der Alkene beschränkt. Auch Alkine oder heteroatomtragende Gruppen, die eine Mehrfachbindung besitzen, können verwendet werden. Wenn es sich um Carbonyle handelt, spricht man auch von einer Carbonyl-En-Reaktion. Insofern zerfällt die Vorläuferverbindung der Formel (I) im Sinne einer Reaktion, welche eine Umkehrung der Carbonyl-En-Reaktion darstellt, in eine Carbonylverbindung (Aldehyd oder Keton) der Formel (III), welche dem Enophil entspricht, sowie in ein Alken mit allylischen Wasserstoff-Atom gemäß Formel (II).

Gemäß der Erfindung ist wenigstens eine der beim Zerfall der Vorläuferverbindung gemäß Formel (I) resultierenden Verbindungen der Formel (II) oder (III) ein Riechstoff. Es kann sich auch bei beiden Zerfallsverbindungen um Riechstoffe handeln. Besonders bevorzugt ist aber, dass beim Zerfall der Vorläuferverbindung ein Riechstoff in Gestalt eines Alkens mit allylischen Wasserstoff-Atom gemäß der Formel (II) resultiert.

Somit ermöglicht die vorliegende Erfindung die gezielte Freisetzung von duftenden Aldehyden, -ketonen (duftenden Aldehyden, -ketonen im Sinne von Riechstoffen) und/oder Riech- oder Aromastoffen mit allylischem Wasserstoffatom. Die Freisetzung kann insbesondere thermisch induziert werden, beispielsweise bei der Anwendung eines Wäschetrockners, beim Bügeln oder bei der Maschinenwäsche vorzugsweise bei hohen Temperaturen.

Die Freisetzung kann auch säurekatalytisch induziert werden, so dass die vorliegende Erfindung auch die verzögerte Freisetzung von duftenden Aldehyden, -ketonen und/oder Riech- oder Aromastoffen mit allylischem Wasserstoffatom durch pH-Änderung ermöglicht, beispielsweise durch Absenken des pH-Wertes im Spülgang oder schleichend auf trockener Wäsche durch Kontakt mit der stets schwach sauren Umgebungsfeuchtigkeit.

Durch die beiden Freisetzungsmechanismen, die sich gewünschtenfalls auch kombinieren lassen, kann sowohl ein sogenannter "Duft-Boost" (Erzeugen eines starken Dufteindruckes) gezielt hervorgerufen werden, z.B. beim Bügeln. Ebenso kann auch eine verlängerte Duftwirkung realisiert werden.

Mit Blick auf Nahrungsmittel und deren Zubereitung ermöglicht die vorliegende Erfindung die gezielte Freisetzung von Aromastoffaldehyden,- ketonen und/oder-alkenen durch Temperaturreiz, beispielsweise beim Kochen, Braten oder Backen.

Bevorzugte Beispiele für erfindungsgemäß einsetzbare Allylether sind z.B. der Benzylallylether und dessen Derivate, wie z.B. p-Methoxybenzylallylether.

Ebenfalls bevorzugte Beispiele für erfindungsgemäß einsetzbare Allylether sind z.B. die Alkyl- oder Alkenylallylether, wie z.B. n-Octyl-Allylether, n-Heptyl-Allylether, n-Nonyl-Allylether, n-Decyl-Allylether, 2-Undecyl-Allylether oder 2-Heptyl-6-methyl-5-en-yl-allylether.

Gemäß einer bevorzugten Ausführungsform steht in der Formel (I) der Rest R⁶ also für Wasserstoff und der Rest R⁷ für einen Alkyl- oder Alkenylrest mit wenigstens 7 Kohlenstoffatomen oder für einen gegebenenfalls substituierten Phenylrest.

Gemäß einer bevorzugten Ausführungsform der Erfindung sind in der Formel (I) die Reste R² sowie R⁴ Wasserstoff-Reste (Vorläuferverbindung entspricht also: R¹CH=CR³-CHR³-O-CHR⁶R⁷), mit der Maßgabe, dass R¹, R³ und R⁵ für Reste stehen, die in einer Verbindung der allgemeinen Formel (IV)

R¹CH₂-CR³=CHR⁵ (IV)

einen Riechstoff ergeben, wobei vorzugsweise der Rest R³ mit einem der Reste R¹ oder R⁵ ein Ringsystem bilden kann,
und/oder mit der Maßgabe, dass R⁶ und R⁷ für Reste stehen, die in einer Verbindung der allgemeinen Formel (III)

R⁶R⁷C=O (III)

einen Riechstoff ergeben. Im Sinne einer bevorzugten Ausführungsform bildet dabei der Rest R³ entweder mit dem Rest R¹ oder aber mit dem Rest R⁵ ein Ringsystem und/oder der Rest R⁶ bildet mit dem Rest R⁷ ein Ringsystem.

Gemäß einer weiteren bevorzugten Ausführungsform der Erfindung sind in der Formel (I) die Reste R², R⁴ sowie R⁶ Wasserstoff-Reste (Vorläuferverbindung entspricht also: R¹CH=CR³-CHR⁵-O-CH₂R⁷),
mit der Maßgabe, dass R¹, R³ und R⁵ für Reste stehen, die in einer Verbindung der allgemeinen Formel (IV)

R¹CH₂-CR³=CHR⁵ (IV)

einen Riechstoff ergeben, wobei vorzugsweise der Rest R³ mit einem der Reste R¹ oder R⁵ ein Ringsystem bilden kann,
und/oder mit der Maßgabe, dass R⁷ für einen Rest steht, der in einer Verbindung der allgemeinen Formel (V)

R⁷CHO (V)

einen Riechstoff ergibt. Im Sinne einer bevorzugten Ausführungsform bildet der Rest R³ entweder mit dem Rest R¹ oder aber mit dem Rest R⁵ ein Ringsystem.

Gemäß einer weiteren bevorzugten Ausführungsform der Erfindung sind in der Formel (I) die Reste R², R⁴, R⁵ sowie R⁶ Wasserstoff-Reste (Vorläuferverbindung entspricht also: R¹CH=CR³-CH₂-O-CH₂R⁷),
mit der Maßgabe, dass R¹ und R³ für Reste stehen, die in einer Verbindung der allgemeinen Formel (VI)

R¹CH₂-CR³=CH₂ (VI)

einen Riechstoff ergeben, wobei der Rest R³ mit dem Rest R¹ vorzugsweise ein Ringsystem bilden kann,
und/oder mit der Maßgabe, dass R⁷ für einen Rest steht, der in einer Verbindung der allgemeinen Formel (V)

R⁷CHO (V)

einen Riechstoff ergibt. Ein Beispiel für eine solche Vorläuferverbindung ist der folgende Myrtenolether

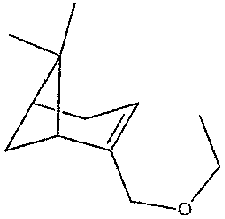

In diesem Beispiel sind die Reste R², R⁴, R⁵ sowie R⁶ Wasserstoff-Reste. Die Reste R¹ und R³ sind miteinander verbunden und bilden ein Ringsystem. Der Rest R⁷ entspricht der Gruppierung CH₃.

Gemäß einer weiteren bevorzugten Ausführungsform der Erfindung sind in der Formel (I) die Reste R¹, R², R⁴ sowie R⁶ Wasserstoff-Reste (Vorläuferverbindung entspricht also: CH₂=CR³-CHR⁵-O-CH₂R⁷),
mit der Maßgabe, dass R³ und R⁵ für Reste stehen, die in einer Verbindung der allgemeinen Formel (VI)

CH₃-CR³=CHR⁵ (VI)

einen Riechstoff ergeben, wobei die Reste R³ und R⁵ vorzugsweise auch ein Ringsystem bilden können,
und/oder mit der Maßgabe, dass R⁷ für einen Rest steht, der in einer Verbindung der allgemeinen Formel (V)

R⁷CHO (V)

einen Riechstoff ergibt. Ein Beispiel für eine solche Vorläuferverbindung ist der Pinocarveyloxy-essigsäuremethylester:

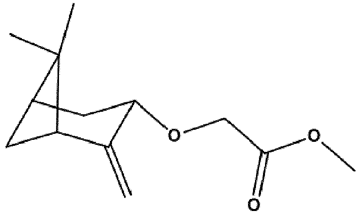

In diesem Beispiel sind die Reste R¹, R², R⁴ sowie R⁶ Wasserstoff-Reste. Die Reste R³ und R⁵ sind miteinander verbunden und bilden ein Ringsystem. Der Rest R⁷ entspricht der Gruppierung COO-Me.

Es ist zwar besonders bevorzugt, dass beim Zerfall der Vorläuferverbindung ein Riechstoff in Gestalt eines Alkens mit allylischen Wasserstoff-Atom gemäß der Formel (II) resultiert. Aber gleichwohl ermöglicht die vorliegende Erfindung auch die Freisetzung duftender Carbonylverbindungen, insbesondere von Riechstoffaldehyden.

Eine weitere bevorzugte Ausführungsform der Erfindung liegt deshalb dann vor, wenn die Verbindung der allgemeinen Formel (III) bzw. (V) ein Riechstoffaldehyd ist, insbesondere ausgewählt aus Adoxal (2,6,10-Trimethyl-9-undecenal), Anisaldehyd (4-Methoxybenzaldehyd), Cymal (3-(4-Isopropylphenyl)-2-methylpropanal), Ethylvanillin, Florhydral (3-(3-isopropylphenyl)butanal]), Helional (3-(3,4-Methylendioxyphenyl)-2-methylpropanal), Heliotropin, Hydroxycitronellal, Lauraldehyd, Lyral (3- und 4-(4-Hydroxy-4-methylpentyl)-3-cyclohexen-1-carboxaldehyd), Methylnonylacetaldehyd, Lilial (3-(4-tert-Butylphenyl)-2-methylpropanal), Phenylacetaldehyd, Undecylenaldehyd, Vanillin, 2,6,10-Trimethyl-9-undecenal, 3-Dodecen-1-al, alpha-n-Amylzimtaldehyd, Melonal (2,6-Dimethyl-5-heptenal), 2,4-Dimethyl-3-cyclohexen-1-carboxaldehyd (Triplal), Benzaldehyd, 3-(4-tert-Butylphenyl)-propanal, 2-Methyl-3-(para-methoxyphenyl)propanal, 2-Methyl-4-(2,6,6-timethyl-2(1)-cyclohexen-1-yl)butanal, 3-Phenyl-2-propenal, cis-/trans-3,7-Dimethyl- 2,6-octadien-1-al, 3,7-Dimethyl-6-octen-1-al, [(3,7-Dimethyl-6-octenyl)oxy]acetaldehyd, 4-Isopropylbenzylaldehyd, 1,2,3,4,5,6,7,8-Octahydro-8,8-dmethyl-2-naphthaldehyd, 2,4-Dimethyl-3-cyclohexen-1-carboxaldehyd, 2-Methyl-3-(isopropylphenyl)propanal, 1-Decanal, 2,6-Dimethyl-5-heptenal, 4-(Tricyclo[5.2.1.0(2,6)]-decyliden-8)-butanal, Octahydro-4,7-methan-1 H-indencarboxaldehyd, 3-Ethoxy-4-hydroxybenzaldehyd, para-Ethyl-alpha,alpha-dimethylhydrozimtaldehyd, alpha-Methyl-3,4-(methylendioxy)-hydrozimtaldehyd, 3,4-Methylendioxybenzaldehyd, alpha-n-Hexylzimtaldehyd, m-Cymen-7-carboxaldehyd, alpha-Methylphenylacetaldehyd, 7-Hydroxy-3,7-dimethyloctanal, Undecenal, 2,4,6-Trimethyl-3-cyclohexen-1-carboxaldehyd, 4-(3)(4-Methyl-3-pentenyl)-3-cyclohexencarboxaldehyd, 1-Dodecanal, 2,4-Dimethylcyclohexen-3-carboxaldehyd, 4-(4-Hydroxy-4-methylpentyl)-3-cylohexen-1-carboxaldehyd, 7-Methoxy-3,7-dimethyloctan-1-al, 2-Methylundecanal, 2-Methyldecanal, 1-Nonanal, 1-Octanal, 2,6,10-Trimethyl-5,9-undecadienal, 2-Methyl- 3-(4-tert-butyl)propanal, Dihydrozimtaldehyd, 1-Methyl-4-(4-methyl-3-pentenyl)-3-cyclohexen-1-carboxaldehyd, 5- oder 6-Methoxyhexahydro-4,7-methanindan-1- oder-2-carboxaldehyd, 3,7-Dimethyloctan-1-al, 1-Undecanal, 10-Undecen-1-al, 4-Hydroxy-3-methoxybenzaldehyd, 1-Methyl-3-(4-methylpentyl)-3-cyclohexencarboxaldehyd, 7-Hydroxy-3,7-dimethyl-octanal, trans-4-Decenal, 2,6-Nonadienal, para-Tolylacetaldehyd, 4-Methylphenylacetaldehyd, 2-Methyl-4-(2,6,6-trimethyl-1-cyclohexen-1-yl)-2-butenal, ortho-Methoxyzimtaldehyd, 3,5,6-Trimethyl-3-cyclohexencarboxaldehyd, 3,7-Dimethyl-2-methylen-6-octenal, Phenoxyacetaldehyd, 5,9-Dimethyl-4,8-decadienal, Päonienaldehyd (6,10-Dimethyl-3-oxa-5,9-undecadien-1-al), Hexahydro- 4,7-methanindan-1-carboxaldehyd, 2-Methyloctanal, alpha-Methyl-4-(1-methylethyl)benzolacetaldehyd, 6,6-Dimethyl-2-norpinen-2-propionaldehyd, para-Methylphenoxyacetaldehyd, 2-Methyl-3-phenyl-2-propen- 1-al 3,5,5-Trimethylhexanal, Hexahydro-8,8-dimethyl-2-naphthaldehyd, 3-Propylbicyclo[2.2.1]-hept-5-en-2-carbaldehyd, 9-Decenal, 3-Methyl-5-phenyl-1-pentanal, Methylnonylacetaldehyd, Hexanal sowie trans-2-Hexenal.

Für weitere geeignete Riechstoffe, ausgewählt aus Aldehyden, wird auf Steffen Arctander Published 1960 and 1969 respectively, Reprinted 2000 ISBN: Aroma Chemicals Vol. 1: 0-931710-37-5, Aroma Chemicals Vol. 2: 0-931710-38-3, verwiesen. Geeignete Riechstoffe, ausgewählt aus Ketonen, sind ebenfalls diese Literaturstelle zu entnehmen.

Gemäß einer weiteren bevorzugten Ausführungsform der Erfindung ist das Alken gemäß Formel (II), (IV) bzw. (VI) ausgewählt aus Riech- oder Aromastoffen mit allylischem Wasserstoffatom, insbesondere aus Limonen, α-Phellandren, β-Phellandren, α-Pinen, β- Pinen, Camphen, Caryophyllen, Longifolen, Ocimen, α- Terpinen, β-Terpinen, γ-Terpinen, α-Terpineol, δ-Terpineol, γ-Terpineol, β-Terpineol, α-Citronellol, β- Citronellol, α-Citronellal, β- Citronellal, Linalool, Geraniol, Santalol, Hasmigone, Carvon, 2-Caren, 3-Caren, 4-Caren, Elemol und/oder Curcumen. Insbesondere bevorzugt sind solche Alkene gemäß Formel (II), (IV) bzw. (VI), bei welchen es sich um echte Kohlenwasserstoffe handelt, d.h. nur aus Kohlenstoff (C) und Wasserstoff (H) bestehende Verbindungen, wie z. B. Pinene oder Terpinene. Weitere geeignete Riechstoffe mit allylischem Wasserstoffatom können der bereits genannten Literaturstelle, Steffen Arctander, entnommen werden.

Die Freisetzung der Riech- oder Aromastoffe aus der Vorläuferverbindung wird insbesondere durch Wärmezufuhr und/oder durch Säurekatalyse induziert, wobei der Einsatz von Brønsted-Säuren bevorzugt, aber auch der Einsatz von Lewis-Säuren möglich ist. Die Säuren können in katalytischen Mengen eingesetzt werden. Insbesondere ist eine Wärmezufuhr in Verbindung mit dem Einsatz katalytischer Mengen an Säuren bevorzugt.

Riechstoffvorläufer im Sinne dieser Erfindung gemäß der Formel (I) mit Ringbildung zwischen den Resten R⁶ und R⁷, sind besonders bevorzugt, da bei diesen Verbindungen die Aktivierungsenergie für die thermische Spaltung besonders klein ist.

Die erfindungsgemäßen Riechvorläufer eignen sich sehr gut zur Einarbeitung in andere Kompositionen bzw. Mittel, wie z.B. Parfüm- oder Aromastoffzusammensetzungen, Waschmittel usw. und ermöglichen im Rahmen der Anwendung des Mittels eine gezielte Duft- bzw. Aromafreisetzung. Die Riechvorläufer sind in solche Mittel stabil einarbeitbar. Die resultierenden Mittel sind lagerstabil.

Eine erfindungsgemäße Riechstoffzusammensetzung enthält neben der erfindungsgemäßen Vorläuferverbindung vorzugsweise noch weitere in Parfümen bzw. Riechstoff- oder Aromastoffzusammensetzungen gebräuchliche Stoffe. Dazu zählen insbesondere Riechstoffe bzw. Aromastoffe und/oder Lösemittel.

Gemäß einer bevorzugten Ausführungsform der Erfindung enthält die erfindungsgemäße Riechstoffzusammensetzung mindestens eine erfindungsgemäße Vorläuferverbindung, wie zuvor beschrieben, in Mengen zwischen 0,001 und 25 Gew.-%, vorzugsweise zwischen 0,01 und 15 Gew.-%, in weiter vorteilhafter Weise zwischen 0,1 und 10 Gew.-%, insbesondere zwischen 1 und 5 Gew.-%, jeweils bezogen auf die gesamte Zusammensetzung.

Gemäß einer besonders bevorzugten Ausführungsform der Erfindung enthält die erfindungsgemäße Riechstoffzusammensetzung neben der erfindungsgemäßen Vorläuferverbindung zusätzliche Riechstoffe, z.B. in einer Menge von 0,1 bis 99 Gew.-%, vorzugsweise 5-90 Gew.-%, insbesondere 15-70 Gew.-%, bezogen auf die gesamte Riechstoffzusammensetzung.

Ebenfalls können typische Hilfsstoffe enthalten sein, wie z.B. Antioxidationsmittel (Sammelbezeichnung für Verbindungen verschiedenartiger chemischer Struktur, die unerwünschte, durch Sauerstoff-Einwirkung und andere oxidative Prozesse bedingte Veränderungen in den zu schützenden Zusammensetzungen hemmen oder verhindern), Konservierungsmittel (Sammelbezeichnung für Verbindungen verschiedenartiger chemischer Struktur, die unerwünschte, durch Einwirkung von Mikroorganismen oder Kleinlebewesen bedingte Veränderungen in den zu schützenden Zusammensetzungen hemmen oder verhindern) oder z.B. Fixateure.

Fixateure, die als Hilfsstoffe optional einsetzbar sind, sind Stoffe, die Riechstoffen eine erhöhte Beständigkeit verleihen können. Als Fixateure sind insbesondere geeignet die sogenannten Eigenfixateure, die aufgrund ihrer Schwerflüchtigkeit ihren Eigengeruch lange bewahren, ohne dabei andere, leichter flüchtige Komponenten in ihrer Geruchsentfaltung zu behindern, wie insbesondere die synthetischen Moschuskörper, weiterhin die sogenannten Pseudofixateure als schwachriechende Stoffe, wie z.B. Diethylenglycolmethylether, sowie ferner die durch Adsorptionskräfte fixierenden Fixateure, wie insbesondere Extrakte aus Labdanum, Styrax, Tolubalsam, Benzoe, Iris, Eichenmoos oder Opopanax usw..

Geeignete optionale Lösungsmittel, welche in einer erfindungsgemäßen Riechstoffzusammensetzung vorzugsweise enthalten sein können, sind insbesondere die in der Parfümerie gebräuchlichen, wie vorzugsweise Dipropylengylcol, Diethylenglycol, Isopropylmyristat, Ethanol, Wasser, Propylenglycol und/oder Rizinusöl. Andere geeignete optionale Hilfsstoffe sind z.B. Komplexbildner.

Gemäß einer bevorzugten Ausführungsform der Erfindung enthält die erfindungsgemäße Riechstoffzusammensetzung Lösungsmittel, z.B. in einer Menge von 0,1 bis 95 Gew.-%, vorzugsweise 5-90 Gew.-%, insbesondere 15-70 Gew.-%, bezogen auf die Riechstoffzusammensetzung.

Insbesondere im Falle einer pulverförmigen Riechstoffzusammensetzung können diese als Hilfsstoffe auch feste Trägerstoffe umfassen. Geeignete feste Trägerstoffe sind dem Fachmann bekannt und orientieren sich am Einsatzzweck der Zusammensetzung. Gebräuchliche feste Trägerstoffe für Aromastoffe sind z.B. die Maltodextrine, da diese nahezu geschmacksneutral sind.

Grundsätzlich können die erfindungsgemäßen Riechstoffzusammensetzungen als optionale Hilfsstoffe auch Tenside enthalten. Es ist jedoch besonders bevorzugt, dass, sofern überhaupt Tenside enthalten sind, die erfindungsgemäße Riechstoffzusammensetzung < 15 Gew.-%, vorzugsweise < 5 Gew.-%, insbesondere < 1 Gew.-% Tenside umfasst. Der Tensidgehalt kann auch unter 10 Gew.-% oder unter 3 Gew.-% oder unter 0,5 Gew.-%, unter 0,1 Gew.-% oder unter 0,01 Gew.-% liegen. Wenn Tenside enthalten sind, was optional ist, dann kann eine geeignete Mindestmenge z.B. bei 0,0001 Gew.-% oder 0,001 Gew.-% liegen, Gew.-% jeweils bezogen auf die gesamte Zusammensetzung. Unter den Begriff der Tenside fallen im Sinne der Erfindung auch die Emulgatoren als grenzflächenaktive Stoffe. Bevorzugt einsetzbare Emulgatoren sind ethoxylierte Fettalkohole, ethoxylierte Triglyceride, Sorbitanfettsäureester, sowie hydriertes, ethoxyliertes Rizinusöl.

Gemäß einer weiteren bevorzugten Ausführungsform der Erfindung ist die erfindungsgemäße Riechstoffzusammensetzung flüssig. Sie kann grundsätzlich auch fest sein, was einer weiteren bevorzugten Ausführungsform der Erfindung entspricht.

Die weiteren Riechstoffe, die in der erfindungsgemäßen Riechstoffzusammensetzung optional enthalten sein können, sind keinen besonderen Beschränkungen unterworfen. So können einzelne Riechstoffverbindungen natürlichen oder synthetischen Ursprungs, z.B. vom Typ der Ester, Ether, Aldehyde, Ketone, Alkohole und Kohlenwasserstoffe verwendet werden. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat (DMBCA), Phenylethylacetat, Benzylacetat, Ethylmethylphenylglycinat, Allylcyclohexylpropionat, Styrallylpropionat, Benzylsalicylat, Cyclohexylsalicylat, Floramat, Melusat und Jasmacyclat. Zu den Ethern zählen beispielsweise Benzylethylether und Ambroxan, zu den Aldehyden z.B. die linearen Alkanale mit 8 - 18 C-Atomen, Citral, Citronellal, Citronellyloxyacetaldehyd, Cyclamenaldehyd (3-(4-propan-2-ylphenyl)butanal), Lilial und Bourgeonal, zu den Ketonen z.B. die Jonone, α-Isomethylionon und Methylcedrylketon, zu den Alkoholen Anethol, Citronellol, Eugenol, Geraniol, Linalool, Phenylethylalkohol und Terpineol, zu den Kohlenwasserstoffen gehören hauptsächlich Terpene wie Limonen und Pinen. Bevorzugt werden jedoch Mischungen verschiedener Riechstoffe verwendet, die gemeinsam eine ansprechende Duftnote erzeugen. Die erfindungsgemäßen Zusammensetzungen können auch natürliche Riechstoffgemische enthalten, wie sie aus pflanzlichen Quellen zugänglich sind, z.B. Pine-, Citrus-, Jasmin-, Patchouly-, Rosen- oder Ylang-Ylang-Öl. Ebenfalls geeignet sind Muskateller-Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliöl, Orangenschalenöl und Sandelholzöl.

Weitere herkömmliche Riechstoffe, die im Rahmen der vorliegenden Erfindung in den erfindungsgemäßen Riechstoffzusammensetzungen enthalten sein können, sind beispielsweise die ätherischen Öle wie Angelikawurzelöl, Anisöl, Arnikablütenöl, Basilikumöl, Bayöl, Champacablütenöl, Edeltannenöl, Edeltannenzapfenöl, Elemiöl, Eukalyptusöl, Fenchelöl, Fichtennadelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Kopaïvabalsamöl, Korianderöl, Krauseminzeöl, Kümmelöl, Kuminöl, Lavendelöl, Lemongrasöl, Limetteöl, Mandarinenöl, Melissenöl, Moschuskörneröl, Myrrhenöl, Nelkenöl, Neroliöl, Niaouliöl, Olibanumöl, Origanumöl, Palmarosaöl, Patchuliöl, Perubalsamöl, Petitgrainöl, Pfefferöl, Pfefferminzöl, Pimentöl, Pine-Öl, Rosenöl, Rosmarinöl, Sandelholzöl, Sellerieöl, Spiköl, Sternanisöl, Terpentinöl, Thujaöl, Thymianöl, Verbenaöl, Vetiveröl, Wacholderbeeröl, Wermutöl, Wintergrünöl, Ylang-Ylang-Öl, Ysop-Öl, Zimtöl, Zimtblätteröl, Zitronellöl, Zitronenöl sowie Zypressenöl sowie Ambrettolid, Ambroxan, α-Amylzimtaldehyd, Anethol, Anisaldehyd, Anisalkohol, Anisol, Anthranilsäuremethylester, Acetophe-non, Benzylaceton, Benzaldehyd, Benzoesäureethylester, Benzophenon, Benzylalkohol, Benzylacetat, Benzylbenzoat, Benzylformiat, Benzylvalerianat, Borneol, Bornylacetat, Boisambrene forte, α-Bromstyrol, n-Decylaldehyd, n-Do-decylaldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon-Dimethylether, Hydroxyzimtaldehyd, Hydroxyzimtalkohol, Indol, Iron, Isoeugenol, Isoeugenolmethyl-ether, Isosafrol, Jasmon, Kampfer, Karvakrol, Karvon, p-Kresolmethylether, Cumarin, p-Methoxy-acetophenon, Methyl-n-amylketon, Methylanthranilsäuremethylester, p-Methylacetophenon, Methylchavikol, p-Methylchinolin, Methyl-β-naphthylketon, Methyl-n-nonylacetaldehyd, Methyl-n-nonylketon, Muskon, β-Naphtholethylether, β-Naphthol-methyl-ether, Nerol, n-Nonylaldehyd, Nonylalkohol, n-Octyl-aldehyd, p-Oxy-Acetophenon, Pentadekanolid, β-Phenylethylalkohol, Phenylessigsäure, Pulegon, Safrol, Salicylsäureisoamylester, Salicylsäuremethylester, Salicylsäurehexylester, Salicylsäurecyclohexylester, Santalol, Sandelice, Skatol, Terpineol, Thymen, Thymol, Troenan, γ-Undelacton, Vanilin, Veratrumaldehyd, Zimtaldehyd, Zimatalkohol, Zimtsäure, Zimtsäureethylester, Zimtsäurebenzylester, Diphenyloxid, Limonen, Linalool, Linalylacetat und -propionat, Melusat, Menthol, Menthon, Methyl-n-heptenon, Pinen, Phenylacetaldehyd, Terpinylacetat, Citral, Citronellal und Mischungen daraus.

Gemäß einer besonders bevorzugten Ausführungsform umfasst die erfindungsgemäße Riechstoffzusammensetzung neben einer erfindungsgemäßen Vorläuferverbindung zumindest einen Riechstoff (Duftstoff), zumindest ein Lösungsmittel sowie vorzugsweise zumindest ein Antioxidationsmittel. Besonders geeignete Riechstoffe sind jene, welche in der erfindungsgemäßen Vorläuferverbindung gespeichert sind. Es entspricht somit einer bevorzugten Ausführungsform, wenn die erfindungsgemäße Riechstoffzusammensetzung neben der erfindungsgemäßen Vorläuferverbindung außerdem jenen oder jene Riechstoff(e) enthält, welche(r) in erfindungsgemäßen Vorläuferverbindung gespeichert sind bzw. ist.

Die erfindungsgemäßen Riechstoffzusammensetzungen ermöglichen, wie bereits ausgeführt, Vorteile bei der Beduftung von Verbrauchsprodukten, wie insbesondere Wasch- oder Reinigungsmitteln, da sie bei der Anwendung der Verbrauchsprodukte Duftvorteile ermöglichen, insbesondere mit Blick auf eine gezielte Duft- bzw. Aromafreisetzung sowie auf eine allmähliche Duft- bzw. Aromafreisetzung. Die erfindungsgemäßen Riechstoffzusammensetzungen lassen sich problemlos und stabil in diverse Verbrauchsprodukte wie insbesondere Wasch- oder Reinigungsmittel, Kosmetika, Luftverbesserer, Klebstoffe einarbeiten. Besonders bevorzugte erfindungsgemäße Verbrauchsprodukte sind Wasch- oder Reinigungsmittel. Der Begriff der Wasch- oder Reinigungsmittel umfasst im Sinne dieser Erfindung auch die Wäschenachbehandlungsmittel wie insbesondere Weichspüler, Bügelwasser oder Hygienespüler. Wasch- oder Reinigungsmittel sind dem Fachmann an sich bekannt. Besonders bevorzugte Mittel sind feste, insbesondere pulverförmige Waschmittel, flüssige, insbesondere gelförmige Waschmittel. Die Mittel, wie insbesondere Waschoder Reinigungsmittel, können auch in Form von sogenannten Pouches (also in kleinen Beuteln), in Form von sogenannten Sheets (also Tüchern oder Folien) oder in Tablettenform vorliegen. Die erfindungsgemäßen Parfümzusammensetzungen können vor der Einarbeitung in das Wasch- oder Reinigungsmittel auch verkapselt werden.

Ein weiterer Gegenstand der Erfindung ist ein parfümiertes oder aromatisiertes Verbrauchsprodukt, insbesondere Wasch- oder Reinigungsmittel, Wäschenachbehandlungsmittel, kosmetisches Mittel, Raumbeduftungsmittel, Nahrungsmittel oder Klebstoff, welches eine erfindungsgemäße Vorläuferverbindung, wie zuvor beschrieben, enthält. Das jeweilige Verbrauchsprodukt enthält weiterhin vorteilhafterweise die für das jeweilige Produkt üblichen Bestandteile. Solche Bestandteile sind dem Fachmann grundsätzlich bekannt oder er kann sie der zugehörigen Literatur entnehmen. Besonders vorteilhaft ist der Einsatz der erfindungsgemäßen Parfümzusammensetzungen in Wasch- oder Reinigungsmitteln.

Gegenstand der Erfindung ist demgemäß ein Wasch- oder Reinigungsmittel, enthaltend mindestens einen erfindungsgemäßen Riechstoffvorläufer, wie zuvor beschrieben, in Mengen zwischen 0,0001 und 10 Gew.-%, vorzugsweise zwischen 0,001 und 5 Gew.-%, in weiter vorteilhafter Weise zwischen 0,01 und 3 Gew.-%, insbesondere zwischen 0,1 und 2 Gew.-%, jeweils bezogen auf das gesamte Mittel. Ein solches Mittel enthält weiterhin die für Wasch- oder Reinigungsmittel üblichen Bestandteile, wie z.B. Tenside, welche zum Teil weiter unten noch genauer beschrieben werden.

Bei der Anwendung des Wasch- oder Reinigungsmittel kann die Vorläuferverbindung z.B. beim Waschen auf dem Textil abgelagert werden. Die Duftfreisetzung aus der Vorläuferverbindung kann dann z.B. beim Bügeln erfolgen. Die Temperatur der Bügelsohle eines üblichen Bügeleisens kann bei üblichen Modellen auf über 200°C eingestellt werden, z.B. auf ca. 220 °C, wodurch eine Freisetzung der gewünschten Riechstoffe induziert werden kann. Ebenso ist es möglich, dass die Vorläuferverbindung bereits in der Waschflotte zerfällt, insbesondere wenn bei hohen Temperaturen gewaschen wird, z.B. bei ≥ 60°C oder bei 95°C.

Ein besonders bevorzugtes Wasch- oder Reinigungsmittel im Sinne der Erfindung ist ein Wäschenachbehandlungsmittel, vorzugsweise Weichspüler, Hygienespüler, Dryer-Sheet, Textilerfrischer oder Bügelwasser, enthaltend mindestens einen Riechstoffvorläufer wie zuvor beschrieben, in Mengen zwischen 0,0001 und 10 Gew.-%, vorzugsweise zwischen 0,001 und 5 Gew.-%, in weiter vorteilhafter Weise zwischen 0,01 und 3 Gew.-%, insbesondere zwischen 0,1 und 2 Gew.-%, jeweils bezogen auf das gesamte Mittel. Auch solche Mittel enthalten weiterhin die für die betreffende Produktkategorie üblichen Bestandteile. Beispielsweise enthält ein Hygienespüler üblicherweise Wirkstoffe, die, je nach Einzelfall, ein breites Spektrum an Organismen von Viren, Bakterien, Pilzen abtöten können. Solche Wirkstoffe, wie z.B. Alkylbenzyldimethylammoniumchlorid, sind dem Fachmann an sich bekannt. Beispielsweise enthalten Weichspülerweichmachende Wirkstoffe, in der Regel Kationtenside, vorzugsweise Esterquats, also quartäre Ammonium-Verbindungen mit zwei hydrophoben Resten, die jeweils eine Ester-Gruppe als sogenannte Sollbruchstelle für einen leichteren biologischen Abbau enthalten.

Als besonders vorteilhaft haben sich die erfindungsgemäßen Riechstoffzusammensetzungen und erfindungsgemäßen Wasch- oder Reinigungsmittel, wie insbesondere Wäschennachbehandlungsmittel, bei der Beduftung von Textilien erwiesen.

Ein weiterer Gegenstand der Erfindung ist somit ein Textilbeduftungsverfahren, bei welchem auf ein Textil zumindest ein erfindungsgemäßer Riechstoffvorläufer aufgebracht wird und die Oberfläche des Textils danach Temperaturen von ≥ 50°C, vorzugsweise Temperaturen zwischen 60°C und 250°C ausgesetzt wird, insbesondere in Gegenwart von Säuren, vorzugsweise Brønsted-Säuren. Die Temperatur, welcher man die Oberfläche des Textils zur Freisetzung des Riechstoffs aus der Vorläuferverbindung aussetzt, kann auch mindestens 80°C oder mindestens 95°C betragen. Mit Blick auf die Freisetzung des Riechstoffs beim Bügeln kann die Temperatur auch bei ≥ 120°C, ≥ 150°C oder ≥ 200°C liegen.

Das Aufbringen der Riechstoffvorläufer kann z.B. bei der üblichen Textilwäsche erfolgen, bei welchem sich die Riechstoffvorläufer auf dem Textil ablagern. Das Textil kann aber auch, wie z.B. bei der Applikation von Bügelwasser, einfach mit der betreffenden Zusammensetzung besprüht werden.

Ebenfalls ist es möglich, dass die Spaltung des Riechstoffvorläufers und somit die Freisetzung der gebundenen Riechstoffe bereits während der Textilwäsche, vorzugsweise in einer automatischen Waschmaschine erfolgt, wobei die Freisetzung durch Hitzeeintrag und/oder säurekatalytisch induziert wird.

Ein weiterer Gegenstand der Erfindung ist somit ein Textilwaschverfahren, bei welchem die Waschflotte zumindest einen erfindungsgemäßen Riechstoffvorläufer enthält und bei welchem die Temperatur der Waschflotte im Rahmen des Waschverfahrens auf ≥ 60°C, vorzugsweise auf 80°C insbesondere auf 95°C gebracht wird.

Ebenfalls ist ein weiterer Gegenstand der Erfindung ein Textilwaschverfahren, bei welchem die Waschflotte zumindest einen erfindungsgemäßen Riechstoffvorläufer enthält und bei welchem die Temperatur der Waschflotte im Rahmen des Waschverfahrens ≤ 40°C, vorzugsweise ≤ 30°C und insbesondere ≤ 20°C beträgt. Ein solches Textilwaschverfahren ermöglicht die Ablagerung des erfindungsgemäßen Riechstoffvorläufers auf dem Waschgut, so dass bei der Nachbehandlung des Waschgutes, z.B. beim Bügeln oder bei Einsatz eines automatischen Wäschetrockners eine Freisetzung der gebundenen Riechstoffe erfolgen kann.

Ein weiterer Gegenstand der Erfindung ist ein Textilbeduftungsverfahren, bei welchem feuchte Textilien zusammen mit mindestens einem erfindungsgemäßen Riechstoffvorläufer in einen maschinellen Wäschetrockner eingebracht werden und im Rahmen der maschinellen Wäschetrocknung die Temperatur in der Trockenkammer auf ≥ 60°C, vorzugsweise auf 80°C insbesondere auf 95°C gebracht wird. Dabei kann der Riechstoffvorläufer z.B. bereits durch die Vorbehandlung auf dem feuchten Textil abgelagert sein oder der Riechstoffvorläufer kann z.B. über ein Trocknertuch in den Wäschetrockner eingebracht werden.

Ein weiterer Gegenstand der Erfindung ist ein Textilbeduftungsverfahren, bei welchem auf ein Textil ein erfindungsgemäßer Riechstoffvorläufer aufgebracht wird und danach der pH-Wert des Textils oder des das Textil umgebenden Mediums auf kleiner 7, vorzugsweise kleiner 5 gebracht wird. Dieses Verfahren umfasst die sukzessive Spaltung der auf dem Textil abgelagerten Vorläuferverbindung durch die stets saure Umgebungsluft.

Ein weiterer Gegenstand der Erfindung ist ein Aromagebungsverfahren bei der Nahrungszubereitung, bei welchem ein Nahrungsmittel (z.B. ein Teig aus Getreide-Mahlprodukten), ein Nahrungsmittelvorprodukt (z.B. ein Backmischung) oder ein Hilfsmittel der Nahrungsmittelzubereitung (z.B. ein Backpulver), welches einen erfindungsgemäßen Riechstoffvorläufer enthält, erhitzt wird, so dass es Temperaturen > 50°C (vorzugsweise > 100°C, insbesondere > 180°C) ausgesetzt ist, insbesondere im Rahmen nahrungsmittelzubereitungsüblicher Vorgänge wie Kochen, Braten, Mikrowellengaren oder Backen.

Bevorzugt enthalten erfindungsgemäße Wasch oder Reinigungsmittel bzw. Wäschenachbehandlungsmittel neben der erfindungsgemäßen Vorläuferverbindung wenigstens eine, vorzugsweise mehrere, aktive Komponenten, insbesondere wasch-, pflege- und/oder reinigungsaktive Komponenten, vorteilhafterweise ausgewählt aus der Gruppe umfassend anionische Tenside, kationische Tenside, amphotere Tenside, nichtionische Tenside, Acidifizierungsmittel, Alkalisierungsmittel, Anti-Knitter-Verbindungen, antibakterielle Stoffe, Antioxidantien, Antiredepositionsmittel, Antistatika, Buildersubstanzen (Gerüststoffe), Bleichmittel, Bleichaktivatoren, Bleichstabilisatoren, Bleichkatalysatoren, Bügelhilfsmittel, Riechstoffe, Einlaufverhinderer, Elektrolyte, Enzyme, Farbschutzstoffe, Farbstoffe, Farbübertragungsinhibitoren, Fluoreszensmittel, Fungizide, Germizide, geruchskomplexierende Substanzen, Hydrotrope, Klarspüler, Komplexbildner, Konservierungsmittel, Korrosionsinhibitoren, optische Aufheller, Perlglanzgeber, pH-Stellmittel, Phobier- und Imprägniermittel, Polymere, Quell- und Schiebefestmittel, Schauminhibitoren, Schichtsilikate, schmutzabweisende Stoffe, Silberschutzmittel, Silikonöle, UV-Schutz-Substanzen, Viskositätsregulatoren, Verdickungsmittel, Verfärbungsinhibitoren, Vergrauungsinhibitoren, Vitamine und/oder Avivage-Wirkstoffe.

Die Mengen der weiteren möglichen Inhaltsstoffe in den erfindungsgemäßen Wasch- oder Reinigungsmitteln bzw. Wäschenachbehandlungsmitteln orientieren sich jeweils am Einsatzzweck der betreffenden Mittel und der Fachmann ist mit den Größenordnungen der einzusetzenden Mengen der optionalen Inhaltsstoffe grundsätzlich vertraut oder kann diese der zugehörigen Fachliteratur entnehmen.

Je nach Einsatzzweck der erfindungsgemäßen Wasch- oder Reinigungsmittel bzw. Wäschenachbehandlungsmittel wird man beispielsweise den Tensidgehalt höher oder niedriger wählen. Üblicherweise liegt z. B. der Tensidgehalt beispielsweise von Waschmitteln zwischen z.B. 5 und 50 Gew.-%, vorzugsweise zwischen 10 und 30 Gew.-% und insbesondere zwischen 15 und 25 Gew.-%, während Reinigungsmittel für das maschinelle Geschirrspülen üblicherweise zwischen z.B. 0,1 und 10 Gew.-%, vorzugsweise zwischen 0,5 und 7,5 Gew.-% und insbesondere zwischen 1 und 5 Gew.-% Tenside enthalten.

Die erfindungsgemäßen Wasch- oder Reinigungsmittel bzw. Wäschenachbehandlungsmittel können vorzugsweise Tenside enthalten, wobei insbesondere anionische Tenside, nichtionische Tenside und deren Gemische, aber auch kationische Tenside in Frage kommen.

Zu den optional einsetzbaren nichtionischen Tensiden gehören die Alkoxylate, insbesondere die Ethoxylate und/oder Propoxylate, von gesättigten oder ein- bis mehrfach ungesättigten linearen oder verzweigtkettigen Alkoholen mit 10 bis 22 C-Atomen, vorzugsweise 12 bis 18 C-Atomen. Der Alkoxylierungsgrad der Alkohole liegt dabei in der Regel zwischen 1 und 20, vorzugsweise zwischen 3 und 10. Sie können in bekannter Weise durch Umsetzung der entsprechenden Alkohole mit den entsprechenden Alkylenoxiden hergestellt werden. Geeignet sind insbesondere die Derivate der Fettalkohole, obwohl auch deren verzweigtkettige Isomere, insbesondere sogenannte Oxoalkohole, zur Herstellung verwendbarer Alkoxylate eingesetzt werden können. Brauchbar sind demgemäß die Alkoxylate, insbesondere die Ethoxylate, primärer Alkohole mit linearen, insbesondere Dodecyl-, Tetradecyl-, Hexadecyl- oder Octadecyl-Resten sowie deren Gemische. Außerdem sind entsprechende Alkoxylierungsprodukte von Alkylaminen, vicinalen Diolen und Carbonsäureamiden, die hinsichtlich des Alkylteils den genannten Alkoholen entsprechen, verwendbar. Auch kommen die Ethylenoxid- und/oder Propylenoxid-Insertionsprodukte von Fettsäurealkylestern sowie Fettsäurepolyhydroxyamide, in Betracht.

Zur optionalen Einarbeitung in die erfindungsgemäßen Mittel geeignete sogenannte Alkylpolyglykoside sind Verbindungen der allgemeinen Formel (G)ₙ-OR⁸, in der R⁸ einen Alkyl- oder Alkenylrest mit 8 bis 22 C-Atomen, G eine Glykoseeinheit und n eine Zahl zwischen 1 und 10 bedeuten. Bei der Glykosidkomponente (G)ₙ handelt es sich um Oligo- oder Polymere aus natürlich vorkommenden Aldose- oder Ketose-Monomeren, zu denen insbesondere Glucose, Mannose, Fruktose, Galaktose, Talose, Gulose, Altrose, Allose, Idose, Ribose, Arabinose, Xylose und Lyxose gehören. Die aus derartigen glykosidisch verknüpften Monomeren bestehenden Oligomere werden außer durch die Art der in ihnen enthaltenen Zucker durch deren Anzahl, den sogenannten Oligomerisierungsgrad, charakterisiert. Der Oligomerisierungsgrad n nimmt als analytisch zu ermittelnde Größe im allgemeinen gebrochene Zahlenwerte an; er liegt bei Werten zwischen 1 und 10, bei den vorzugsweise eingesetzten Glykosiden unter einem Wert von 1,5, insbesondere zwischen 1,2 und 1,4. Bevorzugter Monomer-Baustein ist wegen der guten Verfügbarkeit Glucose. Der Alkyl- oder Alkenylteil R⁸ der Glykoside stammt bevorzugt ebenfalls aus leicht zugänglichen Derivaten nachwachsender Rohstoffe, insbesondere aus Fettalkoholen, obwohl auch deren verzweigtkettige Isomere, insbesondere sogenannte Oxoalkohole, zur Herstellung verwendbarer Glykoside eingesetzt werden können. Brauchbar sind demgemäß insbesondere die primären Alkohole mit linearen Octyl-, Decyl-, Dodecyl-, Tetradecyl-, Hexadecyl- oder Octadecylresten sowie deren Gemische. Besonders bevorzugte Alkylglykoside enthalten einen Kokosfettalkylrest, das heißt Mischungen mit im wesentlichen R⁸=Dodecyl und R⁸=Tetradecyl.

Nichtionisches Tensid ist in erfindungsgemäßen Wasch- oder Reinigungsmitteln vorzugsweise in Mengen von 0,1 Gew.-% bis 30 Gew.-%, insbesondere von 1 Gew.-% bis 25 Gew.-% optional enthalten, Gew.-% bezogen auf das gesamte Wasch- oder Reinigungsmittel.

Die Wasch- oder Reinigungsmittel können stattdessen oder zusätzlich weitere optionale Tenside enthalten, vorzugsweise Aniontenside.

Vorzugsweise sind Aniontenside des Sulfat- oder Sulfonat-Typs, in Mengen von vorzugsweise nicht über 30 Gew.-%, insbesondere von 0,1 Gew.-% bis 18 Gew.-%, jeweils bezogen auf gesamtes Wasch- oder Reinigungsmittel, optional enthalten. Als für den Einsatz in den erfindungsgemäßen Wasch- oder Reinigungsmittel besonders geeignete Aniontenside sind die Alkyl- und/oder Alkenylsulfate mit 8 bis 22 C-Atomen, die ein Alkali-, Ammonium- oder Alkyl- beziehungsweise Hydroxyalkyl-substituiertes Ammoniumion als Gegenkation tragen, zu nennen. Bevorzugt sind die Derivate der Fettalkohole mit insbesondere 12 bis 18 C-Atomen und deren verzweigtkettiger Analoga, der sogenannten Oxoalkohole. Die Alkyl- und Alkenylsulfate können in bekannter Weise durch Reaktion der entsprechenden Alkoholkomponente mit einem üblichen Sulfatierungsreagenz, insbesondere Schwefeltrioxid oder Chlorsulfonsäure, und anschließende Neutralisation mit Alkali-, Ammonium- oder Alkyl- beziehungsweise Hydroxyalkyl-substituierten Ammoniumbasen hergestellt werden. Derartige Alkyl- und/oder Alkenylsulfate sind in den Wasch- oder Reinigungsmitteln vorzugsweise in Mengen von 0,1 Gew.-% bis 20 Gew.-%, insbesondere von 0,5 Gew.-% bis 18 Gew.-% optional enthalten.

Zu den einsetzbaren Tensiden vom Sulfat-Typ gehören auch die sulfatierten Alkoxylierungsprodukte der genannten Alkohole, sogenannte Ethersulfate. Vorzugsweise enthalten derartige Ethersulfate 2 bis 30, insbesondere 4 bis 10, Ethylenglykol-Gruppen pro Molekül. Zu den einsetzbaren Aniontensiden vom Sulfonat-Typ gehören die durch Umsetzung von Fettsäureestern mit Schwefeltrioxid und anschließender Neutralisation erhältlichen α-Sulfoester, insbesondere die sich von Fettsäuren mit 8 bis 22 C-Atomen, vorzugsweise 12 bis 18 C-Atomen, und linearen Alkoholen mit 1 bis 6 C-Atomen, vorzugsweise 1 bis 4 C-Atomen, ableitenden Sulfonierungsprodukte, sowie die durch formale Verseifung aus diesen hervorgehenden Sulfofettsäuren.

Besonders bevorzugt optional einsetzbare Aniontenside sind die Alkylbenzolsulfonate, wie z.B. Natrium-Dodecylbenzolsulfonat.

Anionisches Tensid ist in erfindungsgemäßen Wasch- oder Reinigungsmitteln vorzugsweise in Mengen von 0,1 Gew.-% bis 30 Gew.-%, insbesondere von 1 Gew.-% bis 25 Gew.-% optional enthalten, Gew.-% bezogen auf das gesamte Wasch- oder Reinigungsmittel.

Als weitere optional einsetzbare tensidische Inhaltsstoffe der Wasch- oder Reinigungsmittel kommen Seifen in Betracht, wobei gesättigte Fettsäureseifen, wie die Salze der Laurinsäure, Myristinsäure, Palmitinsäure oder Stearinsäure, sowie aus natürlichen Fettsäuregemischen, zum Beispiel Kokos-, Palmkern- oder Talgfettsäuren, abgeleitete Seifen geeignet sind. Insbesondere sind solche Seifengemische bevorzugt, die zu 50 Gew.-% bis 100 Gew.-% aus gesättigten C₁₂-C₁₈-Fettsäureseifen und zu bis 50 Gew.-% aus Ölsäureseife zusammengesetzt sind. Vorzugsweise ist Seife in dem erfindungsgemäßen Wasch- oder Reinigungsmitteln in Mengen von 0,1 Gew.-% bis 5 Gew.-% optional enthalten. Insbesondere in flüssigen Wasch- oder Reinigungsmitteln können jedoch auch höhere Seifenmengen von bis zu 20 Gew.-% optional enthalten sein.

Auch Kationtenside können optional in den erfindungsgemäßen Wasch- oder Reinigungsmitteln, insbesondere in den erfindungsgemäßen Wäschenachbehandlungsmittel enthalten sein. Beispiele für Kationtenside sind quartäre Ammonium-Verbindungen mit vorzugsweise einem oder insbesondere zwei hydrophoben Alkyl-Resten. Besonders bevorzugt sind Esterquats, also quartäre Ammonium-Verbindungen mit zwei hydrophoben Resten, die jeweils eine Ester-Gruppe als sogenannte Sollbruchstelle für einen leichteren biologischen Abbau enthalten. Bevorzugt einsetzbare Esterquats sind Methyl-N-(2-hydroxyethyl)-N,N-di(talgacyloxyethyl)ammonium-methosulfat, Bis-(palmitoyloxyethyl)-hydroxyethyl-methyl-ammonium-methosulfat, 1,2-Bis-[talgacyloxy]-3-trimethyl-ammoniumpropanchlorid, N,N-Dimethyl-N,N-di(talgacyloxyethyl)ammonium-methosulfat oder Methyl-N,N-bis(stearoyloxyethyl)-N-(2-hydroxyethyl)ammonium-methosulfat.

Die kationischen Tenside sind in den erfindungsgemäßen Mitteln optional in Mengen von vorzugsweise 0,05 bis 20 Gew.%, bezogen auf das gesamte Mittel, enthalten. Mengen von 0,1 bis 5 Gew.% sind besonders bevorzugt.

Gemäß einer bevorzugten Ausführungsform der Erfindung sind Tenside in erfindungsgemäßen Wasch- oder Reinigungsmitteln in einer Gesamtmenge von vorzugsweise 5 Gew.-% bis 50 Gew.-%, insbesondere von 8 Gew.-% bis 30 Gew.-%, enthalten. Insbesondere in Wäschenachbehandlungsmitteln werden vorzugsweise bis zu 30 Gew.-%, insbesondere 5 Gew.-% bis 15 Gew.-% Tenside, unter diesen bevorzugt wenigstens anteilsweise Kationtenside, eingesetzt.

Ein erfindungsgemäßes Wasch- oder Reinigungsmittel kann vorzugsweise mindestens einen Builder, vorzugsweise einen wasserlöslichen und/oder wasserunlöslichen, organischen und/oder anorganischen Builder enthalten. Bevorzugt ist der Einsatz wasserlöslicher Builder.

Zu den wasserlöslichen organischen Buildersubstanzen gehören Polycarbonsäuren, insbesondere Citronensäure und Zuckersäuren, monomere und polymere Aminopolycarbonsäuren, insbesondere Methylglycindiessigsäure, Nitrilotriessigsäure und Ethylendiamintetraessigsäure sowie Polyasparaginsäure, Polyphosphonsäuren, insbesondere Aminotris(methylenphosphonsäure), Ethylendiamintetrakis(methylenphosphonsäure) und 1-Hydroxyethan-1,1-diphosphonsäure, polymere Hydroxyverbin-dungen wie Dextrin sowie polymere (Poly-)carbonsäuren, polymere Acrylsäuren, Methacrylsäuren, Maleinsäuren und Mischpolymere aus diesen, die auch geringe Anteile polymerisierbarer Substanzen ohne Carbonsäurefunktionalität einpolymerisiert enthalten können. Geeignete, wenn auch weniger bevorzugte Verbindungen dieser Klasse sind Copolymere der Acrylsäure oder Methacrylsäure mit Vinylethern, wie Vinylmethylethern, Vinylester, Ethylen, Propylen und Styrol, in denen der Anteil der Säure mindestens 50 Gew.-% beträgt.

Organische Buildersubstanzen können gewünschtenfalls in Mengen bis zu 40 Gew.-%, insbesondere bis zu 25 Gew.-% und vorzugsweise von 1 Gew.-% bis 8 Gew.-% in den erfindungsgemäßen Wasch- oder Reinigungsmittel enthalten sein. Mengen nahe der genannten Obergrenze werden vorzugsweise in pastenförmigen oder flüssigen, insbesondere wasserhaltigen, erfindungsgemäßen Wasch- oder Reinigungsmitteln eingesetzt. Erfindungsgemäße Wasch- oder Reinigungsmittel wie Wäschenachbehandlungsmittel, wie z.B. Weichspüler, können gegebenenfalls auch frei von organischem Builder sein.

Als wasserlösliche anorganische Buildermaterialien kommen insbesondere Alkalisilikate und Polyphosphate, vorzugsweise Natriumtriphosphat, in Betracht. Als wasserunlösliche, wasserdispergierbare anorganische Buildermaterialien werden insbesondere kristalline oder amorphe Alkalialumosilikate, in Mengen von z.B. bis zu 50 Gew.-%, vorzugsweise nicht über 40 Gew.-% und in flüssigen Mitteln insbesondere von 1 Gew.-% bis 5 Gew.-%, in den erfindungsgemäßen Wasch- oder Reinigungsmitteln optional eingesetzt. Unter diesen sind die kristallinen Natriumalumosilikate in Waschmittelqualität, insbesondere Zeolith A, P und gegebenenfalls X, bevorzugt. Mengen nahe der genannten Obergrenze werden vorzugsweise in festen, teilchenförmigen Mitteln optional eingesetzt. Geeignete Substitute beziehungsweise Teilsubstitute für das genannte Alumosilikat sind kristalline Alkalisilikate, die allein oder im Gemisch mit amorphen Silikaten vorliegen können. Die in den erfindungsgemäßen Wasch- oder Reinigungsmitteln als Gerüststoffe brauchbaren Alkalisilikate weisen vorzugsweise ein molares Verhältnis von Alkalioxid zu SiO₂ unter 0,95, insbesondere von 1:1,1 bis 1:12 auf und können amorph oder kristallin vorliegen. Amorphe Alkalisilikate sind bevorzugt.

Weiterhin ist es im Sinne einer weiteren bevorzugten Ausführungsform bevorzugt, allenfalls geringe Menge wasserunlöslicher Buildermaterialien (wie z.B. Zeolith) einzusetzen, beispielsweise in Mengen von 0-5 Gew.-%, z.B. 0,1 bis 2 Gew.-%, bezogen auf das gesamte Wasch- oder Reinigungsmittel.

Buildersubstanzen sind in den erfindungsgemäßen Wasch- oder Reinigungsmitteln vorzugsweise in Mengen bis zu 60 Gew.-%, insbesondere von 5 Gew.-% bis 40 Gew.-%, optional enthalten. Erfindungsgemäße Wäschenachbehandlungsmittel, wie z.B. Weichspüler, sind vorzugsweise frei von anorganischem Builder.

Als optional einsetzbare Persauerstoffverbindungen kommen insbesondere organische Persäuren beziehungsweise persaure Salze organischer Säuren, wie Phthalimidpercapronsäure, Perbenzoesäure oder Salze der Diperdodecandisäure, Wasserstoffperoxid und unter den Anwendungsbedingungen Wasserstoffperoxid abgebende anorganische Salze, wie Perborat, Percarbonat und/oder Persilikat, in Betracht. Sofern feste Persauerstoffverbindungen eingesetzt werden sollen, können diese in Form von Pulvern oder Granulaten verwendet werden, die auch in im Prinzip bekannter Weise umhüllt sein können. Besonders bevorzugt kann Alkalipercarbonat, Alkaliperborat-Monohydrat oder insbesondere in flüssigen Mitteln Wasserstoffperoxid in Form wäßriger Lösungen, die 3 Gew.-% bis 10 Gew.-% Wasserstoffperoxid enthalten, eingesetzt werden. Falls ein erfindungsgemäßes Wasch- oder Reinigungsmittel Bleichmittel, wie vorzugsweise Persauerstoffverbindungen, enthält, sind diese in Mengen von vorzugsweise bis zu 50 Gew.-%, insbesondere von 5 Gew.-% bis 30 Gew.-%, vorhanden. Der optionale Zusatz geringer Mengen bekannter Bleichmittelstabilisatoren wie beispielsweise von Phosphonaten, Boraten beziehungsweise Metaboraten und Metasilikaten sowie Magnesiumsalzen wie Magnesiumsulfat kann zweckdienlich sein.

Als Bleichaktivatoren können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, optional eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS), Carbonsäureanhydride, insbesondere Phthalsäureanhydrid, acylierte mehrwertige Alkohole, insbesondere Triacetin, Ethylenglykoldiacetat, 2,5-Diacet-oxy-2,5-dihydrofuran und Enolester sowie acetyliertes Sorbitol und Mannitol beziehungsweise deren Mischungen , acylierte Zuckerderivate, insbesondere Pentaacetylglukose (PAG), Pentaacetylfruktose, Tetraacetylxylose und Octaacetyllactose sowie acetyliertes, gegebenenfalls N-alkyliertes Glucamin und Gluconolacton, und/oder N-acylierte Lactame, z.B. N-Benzoylcaprolactam. Hydrophil substituierte Acylacetale und Acyllactame werden ebenfalls bevorzugt eingesetzt. Auch Kombinationen konventioneller Bleichaktivatoren können eingesetzt werden. Derartige Bleichaktivatoren können im üblichen Mengenbereich, vorzugsweise in Mengen von 1 Gew.-% bis 10 Gew.-%, insbesondere 2 Gew.-% bis 8 Gew.-%, bezogen auf gesamtes Mittel, optional enthalten sein.

Als in den Wasch- oder Reinigungsmitteln optional einsetzbare Enzyme kommen insbesondere solche aus der Klasse der Proteasen, Cutinasen, Amylasen, Pullulanasen, Hemicellulasen, Cellulasen, Lipasen, Oxidasen und Peroxidasen sowie deren Gemische in Frage. Besonders geeignet sind aus Pilzen oder Bakterien, wie Bacillus subtilis, Bacillus licheniformis, Streptomyces griseus, Humicola lanuginosa, Humicola insolens, Pseudomonas pseudoalcaligenes oder Pseudomonas cepacia gewonnene enzymatische Wirkstoffe. Die gegebenenfalls verwendeten Enzyme können an Trägerstoffen adsorbiert und/oder in Hüllsubstanzen eingebettet sein, um sie gegen vorzeitige Inaktivierung zu schützen. Sie sind in den erfindungsgemäßen Wasch- oder Reinigungsmitteln, vorzugsweise in Mengen nicht über 5 Gew.-%, insbesondere von 0,2 Gew.-% bis 2 Gew.-%, optional enthalten.

Die Wasch- oder Reinigungsmittel können als optische Aufheller beispielsweise Derivate der Diaminostilbendisulfonsäure beziehungsweise deren Alkalimetallsalze optional enthalten. Geeignet sind zum Beispiel Salze der 4,4'-Bis(2-anilino-4-morpholino-1,3,5-triazinyl-6-amino)stilben-2,2'-disulfonsäure oder gleichartig aufgebaute Verbindungen, die anstelle der Morpholino-Gruppe eine Diethanolaminogruppe, eine Methylaminogruppe, eine Anilinogruppe oder eine 2-Methoxyethylaminogruppe tragen. Weiterhin können Aufheller vom Typ der substituierten Diphenylstyryle anwesend sein, zum Beispiel die Alkalisalze des 4,4'-Bis(2-sulfostyryl)-diphenyls, 4,4'-Bis(4-chlor-3-sulfostyryl)-diphenyls oder 4-(4-Chlorstyryl)-4'-(2-sulfostyryl)-diphenyls. Auch Gemische der vorgenannten Aufheller können verwendet werden.

Zu den optional einsetzbaren Schauminhibitoren gehören beispielsweise Organopolysiloxane und deren Gemische mit mikrofeiner, gegebenenfalls silanierter Kieselsäure sowie Paraffinwachse und deren Gemische mit silanierter Kieselsäure oder Bisfettsäure-alkylendiamiden. Mit Vorteilen können auch Gemische aus verschiedenen Schauminhibitoren verwendet werden, zum Beispiel solche aus Silikonen, Paraffinen oder Wachsen. Vorzugsweise sind die optionalen Schauminhibitoren, insbesondere Silikon- und/oder Paraffin-haltige Schauminhibitoren, an eine granulare, in Wasser lösliche beziehungsweise dispergierbare Trägersubstanz gebunden. Insbesondere sind dabei Mischungen aus Paraffinwachsen und Bistearylethylendiamiden bevorzugt.

Zusätzlich können die Wasch- oder Reinigungsmittel optional auch Komponenten enthalten, welche die Öl- und Fettauswaschbarkeit aus Textilien positiv beeinflussen, sogenannte soil-release-Wirkstoffe. Dieser Effekt wird besonders deutlich, wenn ein Textil verschmutzt wird, das bereits vorher mehrfach mit einem Waschmittel, das diese öl- und fettlösende Komponente enthält, gewaschen wurde. Zu den bevorzugten öl- und fettlösenden Komponenten zählen z.B. nicht-ionische Celluloseetherwie Methylcellulose und Methylhydroxypropylcellulose mit einem Anteil an Methoxyl-Gruppen von 15 bis 30 Gew.-% und an Hydroxypropoxyl-Gruppen von 1 bis 15 Gew.-%, jeweils bezogen auf den nichtionischen Celluloseether, sowie die aus dem Stand der Technik bekannten Polymere der Phthalsäure und/oder der Terephthalsäure bzw. von deren Derivaten mit monomeren und/oder polymeren Diolen, insbesondere Polymere aus Ethylenterephthalaten und/oder Polyethylenglykolterephthalaten oder anionisch und/oder nichtionisch modifizierten Derivaten von diesen.

Die Wasch- oder Reinigungsmittel können optional auch Farbübertragungsinhibitoren, vorzugsweise in Mengen von 0,1 Gew.-% bis 2 Gew.-%, insbesondere 0,1 Gew.-% bis 1 Gew.-%, enthalten, die in einer bevorzugten Ausgestaltung der Erfindung Polymere aus Vinylpyrrolidon, Vinylimidazol, Vinylpyridin-N-Oxid oder Copolymere aus diesen sind. Brauchbar sind sowohl Polyvinylpyrrolidone, N-Vinyl-imidazol/N-Vinylpyrrolidon-Copolymere, Polyvinyloxazolidone, Copolymere auf Basis von Vinylmonomeren und Carbonsäureamiden, pyrrolidongruppenhaltige Polyester und Polyamide, gepfropfte Polyamidoamine und Polyethylenimine, Polymere mit Amidgruppen aus sekundären Aminen, Polyamin-N-Oxid-Polymere, Polyvinylalkohole und Copolymere auf Basis von Acrylamidoalkenylsulfonsäuren.

Die optional einsetzbaren Vergrauungsinhibitoren haben das Vermögen, den von der Textilfaser abgelösten Schmutz in der Flotte suspendiert zu halten. Hierzu sind wasserlösliche Kolloide meist organischer Natur geeignet, beispielsweise Stärke, Leim, Gelatine, Salze von Ethercarbonsäuren oder Ethersulfonsäuren der Stärke oder der Cellulose oder Salze von sauren Schwefelsäureestern der Cellulose oder der Stärke. Auch wasserlösliche, saure Gruppen enthaltende Polyamide sind für diesen Zweck geeignet. Weiterhin lassen sich andere als die obengenannten Stärkederivate verwenden, zum Beispiel Aldehydstärken. Bevorzugt können Celluloseether, wie Carboxymethylcellulose (Na-Salz), Methylcellulose, Hydroxyalkylcellulose und Mischether, wie Methylhydroxyethylcellulose, Methylhydroxypropylcellulose, Methylcarboxymethylcellulose und deren Gemische, beispielsweise in Mengen von 0,1 bis 5 Gew.-%, bezogen auf das Wasch- oder Reinigungsmittel, als optionale Vergrauungsinhibitoren eingesetzt werden.

Zu den in den erfindungsgemäßen Wasch- oder Reinigungsmitteln, insbesondere wenn sie in flüssiger oder pastöser Form vorliegen, optional verwendbaren organischen Lösungsmitteln gehören vorzugsweise Alkohole mit 1 bis 4 C-Atomen, insbesondere Methanol, Ethanol, Isopropanol und tert.-Butanol, Diole mit 2 bis 4 C-Atomen, insbesondere Ethylenglykol und Propylenglykol, sowie deren Gemische und die aus den genannten Verbindungsklassen ableitbaren Ether. Derartige wassermischbare Lösungsmittel können in den erfindungsgemäßen Wasch- oder Reinigungsmitteln vorzugsweise in Mengen von nicht über 30 Gew.-%, insbesondere von 6 Gew.-% bis 20 Gew.-%, optional vorhanden sein.

Zur Einstellung eines gewünschten, sich durch die Mischung der übrigen Komponenten nicht von selbst ergebenden pH-Werts können die erfindungsgemäßen Wasch- oder Reinigungsmittel Säuren, insbesondere Citronensäure, Essigsäure, Weinsäure, Äpfelsäure, Milchsäure, Glykolsäure, Bernsteinsäure, Glutarsäure und/oder Adipinsäure, aber auch Mineralsäuren, insbesondere Schwefelsäure, oder Basen, insbesondere Ammonium- oder Alkalihydroxide, optional enthalten. Derartige pH-Regulatoren können in den erfindungsgemäßen Wasch- oder Reinigungsmitteln vorzugsweise nicht über 20 Gew.-%, insbesondere von 1,2 Gew.-% bis 17 Gew.-%, optional enthalten sein.

Die Herstellung fester erfindungsgemäßer Mittel, wie insbesondere Wasch- oder Reinigungsmittel, kann in im Prinzip bekannter Weise, zum Beispiel durch Sprühtrocknen oder Granulation, erfolgen, wobei z.B. optionale Persauerstoffverbindung und optionaler Bleichkatalysator gegebenenfalls später zugesetzt werden. Der erfindungsgemäß eingesetzte Riechstoffvorläufer wird vorzugsweise zum Ende der Herstellung in das Mittel eingebracht, vorzugsweise durch Aufsprühen, insbesondere zusammen mit weiteren Riechstoffen bzw. mit einem Parfümöl. Zur Herstellung erfindungsgemäßer Mittel, z.B. Wasch- oder Reinigungsmittel, mit erhöhtem Schüttgewicht, insbesondere im Bereich von 650 g/l bis 950 g/l, ist ein einen Extrusionsschritt aufweisendes Verfahren bevorzugt. Die Herstellung flüssiger erfindungsgemäßer Mittel, z.B. Wasch- oder Reinigungsmittel, kann ebenfalls in an sich bekannter Weise erfolgen, wobei der erfindungsgemäß eingesetzte Riechstoffvorläufer vorzugsweise zum Ende der Herstellung in das Mittel, z.B. Wasch- oder Reinigungsmittel, eingebracht wird, insbesondere zusammen mit weiteren Riechstoffen bzw. mit einem Parfümöl.

Gemäß einer bevorzugten Ausführungsform kann die erfindungsgemäße Lehre dazu eingesetzt werden, den Parfümanteil in den betreffenden Mitteln, wie z.B. Wasch- oder Reinigungsmitteln, wie insbesondere Wäschenachbehandlungsmitteln, herabzusetzen, denn durch die Inkorporation der erfindungsgemäßen Riechstoffzusammensetzung kann eine besonders effiziente Parfümierung gewährleistet werden, die sich je nach Wunsch aus der gezielten Duftfreisetzung und/oder einer lange anhaltenden Duftfreisetzung ergibt.

Ein bevorzugtes erfindungsgemäßes Wasch- oder Reinigungsmittel ist ein festes, insbesondere pulverförmiges Waschmittel, das neben der erfindungsgemäßen Riechstoffvorläuferverbindung vorzugsweise Komponenten enthalten kann, die insbesondere ausgewählt sind aus den folgenden:
(a) Aniontenside, wie vorzugsweise Alkylbenzolsulfonat, Alkylsulfat, z.B. in Mengen von vorzugsweise 5-30 Gew.-%
(b) Nichtionische Tenside, wie vorzugsweise Fettalkoholpolyglycolether, Alkylpolyglucosid, Fettsäureglucamid z.B. in Mengen von vorzugsweise 0,5-15 Gew.-%
(c) Gerüststoffe, wie z.B. Polycarboxylat, Natriumcitrat, in Mengen von z.B. 0-70 Gew.-%, vorteilhafterweise 5-60 Gew.-%, vorzugsweise 10-55 Gew.-%, insbesondere 15-40 Gew.-%,
(d) Alkalien, wie z.B. Natriumcarbonat, in Mengen von z.B. 0-35 Gew.-% vorteilhafterweise 1-30 Gew.-%, vorzugsweise 2-25 Gew.-%, insbesondere 5-20 Gew.-%,
(e) Bleichmittel, wie z.B. Natriumperborat oder Natriumpercarbonat, in Mengen von z.B. 0-30 Gew.-% vorteilhafterweise 5-25 Gew.-%, vorzugsweise 10-20 Gew.-%,
(f) Korrosionsinhibitoren, z.B. Natriumsilicat, in Mengen von z.B. 0-10 Gew.-%, vorteilhafterweise 1-6 Gew.-%, vorzugsweise 2-5 Gew.-%, insbesondere 3-4 Gew.-%,
(g) Stabilisatoren, z.B. Phosphonate, vorteilhafterweise 0-1 Gew.-%,
(h) Schauminhibitor, z.B. Seife, Siliconöle, Paraffine vorteilhafterweise 0-4 Gew.-%, vorzugsweise 0,1-3 Gew.-%, insbesondere 0,2-1 Gew.-%,
(i) Enzyme, z.B. Proteasen, Amylasen, Cellulasen, Lipasen, vorteilhafterweise 0-2 Gew.-%, vorzugsweise 0,2-1 Gew.-%, insbesondere 0,3-0,8 Gew.-%,
(j) Vergrauungsinhibitor, z.B. Carboxymethylcellulose, vorteilhafterweise 0-1 Gew.-%,
(k) Verfärbungsinhibitor, z.B. Polyvinylpyrrolidon-Derivate, z.B. 0-2 Gew.-%,
(l) Stellmittel, z.B. Natriumsulfat, vorteilhafterweise 0-20 Gew.-%,
(m) Optische Aufheller, z.B. Stilben-Derivat, Biphenyl-Derivat, vorteilhafterweise 0-0,4 Gew.-%, insbesondere 0,1-0,3 Gew.-%,
(n) ggf. weitere Riechstoffe
(o) ggf. Wasser
(p) ggf. Seife
(q) ggf. Bleichaktivatoren
(r) ggf. Cellulosderivate
(s) ggf. Schmutzabweiser,
Gew.-% jeweils bezogen auf das gesamte Mittel.

In einer besonders bevorzugten Ausführungsform ist das erfindungsgemäße Wasch- oder Reinigungsmittel festförmig, insbesondere teilchenförmig und enthält neben der erfindungsgemäßen Vorläuferverbindung noch 5 Gew.-% bis 55 Gew.-% Gerüststoffe, 2,5 Gew.-% bis 20 Gew.-% Aniontensid, 1 Gew.-% bis 20 Gew.-% Niotensid, 1 Gew.-% bis 25 Gew.-% Bleichmittel, 0,5 Gew.-% bis 8 Gew.-% Bleichaktivator und 0,1 Gew.-% bis 40 Gew.-% Stellmittel, insbesondere Alkalisulfat, sowie bis zu 2 Gew.-%, insbesondere 0,4 Gew.-% bis 1,2 Gew.-% Enzym, vorzugsweise teilchenförmig konfektioniertes Enzym, insbesondere Protease, Lipase, Amylase, Cellulase und/oder Oxidoreduktase. Diese Ausführungsform kann optional auch frei von Bleichmittel und Bleichaktivator sein.

In einer anderen bevorzugten Ausführungsform der Erfindung liegt das erfindungsgemäße Wasch- oder Reinigungsmittel in flüssiger Form vor, vorzugsweise in Gelform. Bevorzugte flüssige Wasch- oder Reinigungsmittel haben Wassergehalte von z.B. 10-95 Gew.-%, vorzugsweise 20-80 Gew.-% und insbesondere 30-70 Gew.-%, bezogen auf das gesamte Mittel. Im Falle von flüssigen Konzentraten kann der Wassergehalt auch besonders gering sein, z.B. ≤ 30 Gew.-%, vorzugsweise ≤ 20 Gew.-%, insbesondere ≤ 15 Gew.-%, wie z.B. 0,1 bis 10 Gew.-%, betragen, Gew.-% jeweils bezogen auf das gesamte Mittel. Die flüssigen Mittel können auch nichtwässrige Lösungsmittel enthalten.

Ein bevorzugtes erfindungsgemäßes Wasch- oder Reinigungsmittel ist ein flüssiges, insbesondere gelförmiges Waschmittel, das neben der erfindungsgemäßen Riechstoffvorläuferverbindung vorzugsweise Komponenten enthalten kann, die vorzugsweise ausgewählt sind aus den folgenden:
- Aniontenside, wie vorzugsweise Alkylbenzolsulfonat, Alkylsulfat, z.B. in Mengen von vorzugsweise 5-40 Gew.-%
- Nichtionische Tenside, wie vorzugsweise Fettalkoholpolyglycolether, Alkylpolyglucosid, Fettsäureglucamid z.B. in Mengen von vorzugsweise 0,5-25 Gew.-%
- Gerüststoffe, wie z.B. Polycarboxylat, Natriumcitrat, vorteilhafterweise 0-25 Gew.-%, vorzugsweise 0,01-10 Gew.-%, insbesondere 0,1-5 Gew.-%,
- Schauminhibitor, z.B. Siliconöle, Paraffine, in Mengen von z.B. 0-10 Gew.-%, vorteilhafterweise 0,1-4 Gew.-%, vorzugsweise 0,2-2 Gew.-%, insbesondere 1-3 Gew.-%,
- Enzyme, z.B. Proteasen, Amylasen, Cellulasen, Lipasen, in Mengen von z.B. 0-3 Gew.-%, vorteilhafterweise 0,1-2 Gew.-%, vorzugsweise 0,2-1 Gew.-%, insbesondere 0,3-0,8 Gew.-%,
- Optische Aufheller, z.B. Stilben-Derivat, Biphenyl-Derivat, in Mengen von z.B. 0-1 Gew.-%, vorteilhafterweise 0,1-0,3 Gew.-%, insbesondere 0,1-0,4 Gew.-%,
- ggf. weitere Riechstoffe
- Wasser
- ggf. Seife, in Mengen von z.B. 0-25 Gew.-%, vorteilhafterweise 1-20 Gew.-%, vorzugsweise 2-15 Gew.-%, insbesondere 5-10 Gew.-%,
- ggf. Lösungsmittel (vorzugsweise Alkohole), vorteilhafterweise 0-25 Gew.-%, vorzugsweise 1-20 Gew.-%, insbesondere 2-15 Gew.-%, Gew.-% jeweils bezogen auf das gesamte Mittel.

Ein besonders bevorzugtes flüssiges Wasch- oder Reinigungsmittel enthält dabei neben der erfindungsgemäßen Riechstoffvorläuferverbindung zumindest Aniontenside in Mengen von 0,5 Gew.-% bis 20 Gew.-%, nichtionische Tenside in Mengen von 1 Gew.-% bis 25 Gew.-%, Gerüststoffe in Mengen von 1 bis 25 Gew.-%, Enzyme sowie Wasser.

Ein weiteres bevorzugtes erfindungsgemäßes Wasch- oder Reinigungsmittel ist ein flüssiger Weichspüler, der neben der erfindungsgemäßen Riechstoffvorläuferverbindung vorzugsweise Komponenten enthalten kann, die ausgewählt sind aus den folgenden:
- Kationische Tenside, wie insbesondere Esterquats, z.B. in Mengen von 5-30 Gew.-%,
- Cotenside, wie insbesondere Glycerolmonostearat, Stearinsäure, Fettalkohole und/oder Fettalkoholethoxylate, z.B. in Mengen von 0-5 Gew.-%, vorzugsweise 0,1-4 Gew.-%,
- Emulgatoren, wie insbesondere Fettaminethoxylate, z.B. in Mengen von 0-4 Gew.-%, vorzugsweise 0,1-3 Gew.-%,
- ggf. weitere Riechstoffe
- ggf. Farbstoffe, vorzugsweise im ppm-Bereich
- Lösemittel, wie insbesondere Wasser, z.B. in Mengen von 60-90 Gew.-%,
Gew.-% jeweils bezogen auf das gesamte Mittel.

### Beispiele

### Beispiel 1: trans-Pinocarveyloxy-essigsäuremethylester

Struktur:

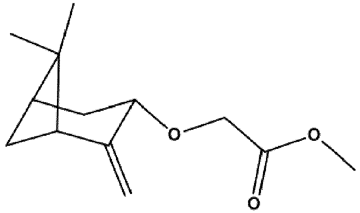

Synthese: In einem mit Argon befüllten Kolben wurden 2 Äquivalente NaH in trockenem THF suspendiert. Anschließend wurde langsam 1 Äquivalent trans-Pinocarveol in trockenem THF zugegeben und 40 Minuten gerührt. Dann wurden 1,5 Äquivalente 2-Bromessigsäuremethylester langsam zugegeben. Nach vollendeter Reaktion wurde der Ansatz mit wenig Wasser versetzt und die wässrige Phase zweimal mit Ethylacetat extrahiert. Die organischen Phasen wurden mit Na₂SO₃- und NaCl - Lösung gewaschen und mit MgSO₄ getrocknet. Überschüssiges Lösungsmittel wurde entfernt und das Rohprodukt säulenchromatographisch gereinigt.

Thermisch induzierte Freisetzung von α-Pinen:
a) Trans-Pinocarveyloxy-essigsäuremethylester wurde in N-Methyl-2-pyrollidon gelöst und für 2h auf 200°C erhitzt. Dabei wandelte sich der trans-Pinocarveyloxy-essigsäuremethylester zu 50% in α-Pinen um, welches per GC-MS nachgewiesen werden konnte.
b) Der trans-Pinocarveyloxy-essigsäuremethylester wurde in ein Rizinusölethoxylat und ätherisches Orangenöl enthaltendes Bügelwasser eingearbeitet. Das Bügelwasser wurde durch Sprühen auf ein Baumwolltuch appliziert, welches danach mit einer Temperatur von 220°C gebügelt wurde. In der Folge konnte ein für das α-Pinen charakteristischer Terpentingeruch wahrgenommen werden.

## Patentansprüche

1. Wasch- oder Reinigungsmittel, enthaltend eine Riechstoffzusammensetzung in Mengen zwischen 0,0001 und 10 Gew.-%, vorzugsweise zwischen 0,001 und 5 Gew.-%, in weiter vorteilhafter Weise zwischen 0,01 und 3 Gew.-%, insbesondere zwischen 0,1 und 2 Gew.-%, jeweils bezogen auf das gesamte Mittel, enthaltend einen Riechstoffvorläufer, bei welchem es sich um einen Allylether der Formel (I) handelt,
R¹R²C=CR³-CR⁴R⁵-O-CHR⁶R⁷ (I),
wobei die Reste R¹, R², R³, R⁴, R⁵, R⁶ und R⁷, unabhängig voneinander jeweils für H oder einen Kohlenwasserstoff-Rest stehen, der acyclisch oder cyclisch, substituiert oder unsubstituiert, verzweigt oder unverzweigt sowie gesättigt oder ungesättigt sein kann, und wobei einzelne Reste R¹, R², R³, R⁴, R⁵,R⁶, R⁷miteinander ein Ringsystem bilden können,
mit der Maßgabe, dass R¹, R², R³, R⁴ und R⁵ für Reste stehen, die in einer Verbindung der allgemeinen Formel (II)
R¹R²CH-CR³=CR⁴R⁵ (II)
einen Riechstoff ergeben,
und/oder mit der Maßgabe, dass R⁶ und R⁷ für Reste stehen, die in einer Verbindung der allgemeinen Formel (III)
R⁶R⁷C=O (III)
einen Riechstoff ergeben.

2. Wasch- oder Reinigungsmittel gemäß Anspruch 1, **dadurch gekennzeichnet, dass** in der Formel (I) die Reste R² sowie R⁴ Wasserstoff-Reste sind,
mit der Maßgabe, dass R¹, R³ und R⁵ für Reste stehen, die in einer Verbindung der allgemeinen Formel (IV)
R¹CH₂-CR³=CHR⁵ (IV)
einen Riechstoff ergeben,
und/oder mit der Maßgabe, dass R⁶ und R⁷ für Reste stehen, die in einer Verbindung der allgemeinen Formel (III)
R⁶R⁷C=O (III)
einen Riechstoff ergeben.

3. Wasch- oder Reinigungsmittel gemäß Anspruch 1, **dadurch gekennzeichnet, dass** in der Formel (I) die Reste R², R⁴ sowie R⁵ Wasserstoff-Reste sind,
mit der Maßgabe, dass R¹, R³ und R⁵ für Reste stehen, die in einer Verbindung der allgemeinen Formel (IV)
R¹CH₂-CR³=CHR⁵ (IV)
einen Riechstoff ergeben, und/oder mit der Maßgabe, dass R⁷ für einen Rest steht, der in einer Verbindung der allgemeinen Formel (V)
R⁷CHO (V)
einen Riechstoff ergibt.

4. Wasch- oder Reinigungsmittel gemäß Anspruch 1, **dadurch gekennzeichnet, dass** in der Formel (I) die Reste R², R⁴, R⁵ sowie R⁶ Wasserstoff-Reste sind,
mit der Maßgabe, dass R¹ und R³ für Reste stehen, die in einer Verbindung der allgemeinen Formel (VI)
R¹CH₂-CR³=CH₂ (VI)
einen Riechstoff ergeben, und/oder mit der Maßgabe, dass R⁷ für einen Rest steht, der in einer Verbindung der allgemeinen Formel (V)
R⁷CHO (V)
einen Riechstoff ergibt.

5. Wasch- oder Reinigungsmittel gemäß Anspruch 1, **dadurch gekennzeichnet, dass** in der Formel (I) die Reste R¹, R², R⁴ sowie R⁶ Wasserstoff-Reste sind,
mit der Maßgabe, dass R³ und R⁵ für Reste stehen, die in einer Verbindung der allgemeinen Formel (VI)
CH₃-CR³=CHR⁵ (VI)
einen Riechstoff ergeben,
und/oder mit der Maßgabe, dass R⁷ für einen Rest steht, der in einer Verbindung der allgemeinen Formel (V)
R⁷CHO (V)
einen Riechstoff ergibt.

6. Wasch- oder Reinigungsmittel gemäß einem der Ansprüche 1 bis 5, **dadurch gekennzeichnet, dass** die Verbindung der allgemeinen Formel (III) bzw. (V) ein Riechstoffaldehyd ist, insbesondere ausgewählt aus Adoxal (2,6,10-Trimethyl-9-undecenal), Anisaldehyd (4-Methoxybenzaldehyd), Cymal (3-(4-Isopropylphenyl)-2-methylpropanal), Ethylvanillin, Florhydral (3-(3-isopropylphenyl)butanal]), Helional (3-(3,4-Methylendioxyphenyl)-2-methylpropanal), Heliotropin, Hydroxycitronellal, Lauraldehyd, Lyral (3- und 4-(4-Hydroxy-4-methylpentyl)-3-cyclohexen-1-carboxaldehyd), Methylnonylacetaldehyd, Lilial (3-(4-tert-Butylphenyl)-2-methylpropanal), Phenylacetaldehyd, Undecylenaldehyd, Vanillin, 2,6,10-Trimethyl-9-undecenal, 3-Dodecen-1-al, alpha-n-Amylzimtaldehyd, Melonal (2,6-Dimethyl-5-heptenal), 2,4-Dimethyl-3-cyclohexen-1-carboxaldehyd (Triplal), Benzaldehyd, 3-(4-tert-Butylphenyl)-propanal, 2-Methyl-3-(para-methoxyphenyl)propanal, 2-Methyl-4-(2,6,6-timethyl-2(1)-cyclohexen-1-yl)butanal, 3-Phenyl-2-propenal, cis-/trans-3,7-Dimethyl-2,6-octadien-1-al, 3,7-Di-methyl-6-octen-1-al, [(3,7-Dimethyl-6-octenyl)oxy]acetaldehyd, 4-Isopropylbenzylaldehyd, 1,2,3,4,5,6,7,8-Octahydro-8,8-dmethyl-2-naphthaldehyd, 2,4-Dimethyl-3-cyclohexen-1-carbox-aldehyd, 2-Methyl-3-(isopropylphenyl)propanal, 1-Decanal, 2,6-Dimethyl-5-heptenal, 4-(Tricyclo[ 5.2.1.0(2,6)]-decyliden-8)-butanal, Octahydro-4,7-methan-1H-indencarboxaldehyd, 3-Ethoxy-4-hydroxybenzaldehyd, para-Ethyl-alpha,alpha-dimethylhydrozimtaldehyd, alpha-Methyl-3,4-(methyl-endioxy)-hydrozimtaldehyd, 3,4-Methylendioxybenzaldehyd, alpha-n-Hexylzimtaldehyd, m-Cy-men-7-carboxaldehyd, alpha-Methylphenylacetaldehyd, 7-Hydroxy-3,7-dimethyloctanal, Undecenal, 2,4,6-Trimethyl-3-cyclohexen-1-carboxaldehyd, 4-(3)(4-Methyl-3-pentenyl)-3-cyclohexencarboxaldehyd, 1-Dodecanal, 2,4-Dimethylcyclohexen-3-carboxaldehyd, 4-(4-Hydroxy-4-methylpentyl)-3-cylohexen-1-carboxaldehyd, 7-Methoxy-3,7-dimethyloctan-1-al, 2-Methylundecanal, 2-Methyldecanal, 1-Nonanal, 1-Octanal, 2,6,10-Trimethyl-5,9-undecadienal, 2-Methyl-3-(4-tert-butyl)propanal, Dihydrozimtaldehyd, 1-Methyl-4-(4-methyl-3-pentenyl)-3-cyclohexen-1-carboxaldehyd, 5- oder 6-Methoxyhexahydro-4,7-methanindan-1- oder -2-carboxaldehyd, 3,7-Dimethyloctan-1-al, 1-Undecanal, 10-Undecen-1-al, 4-Hydroxy-3-methoxybenzaldehyd, 1-Methyl-3-(4-methylpentyl)-3-cyclohexencarboxaldehyd, 7-Hydroxy-3,7-dimethyl-octanal, trans-4-Decenal, 2,6-Nonadienal, para-Tolylacetaldehyd, 4-Methylphenylacetaldehyd, 2-Methyl-4-(2,6,6-trimethyl-1-cyclohexen-1-yl)-2-butenal, ortho-Methoxyzimtaldehyd, 3,5,6-Trimethyl-3-cyclohexencarboxaldehyd, 3,7-Dimethyl-2-methylen-6-octenal, Phenoxyacetaldehyd, 5,9-Dimethyl-4,8-decadienal, Päonienaldehyd (6,10-Dimethyl-3-oxa-5,9-undecadien-1-al), Hexahydro-4,7-methanindan-1-carboxaldehyd, 2-Methyloctanal, alpha-Methyl-4-(1-methylethyl)benzolacetaldehyd, 6,6-Dimethyl-2-norpinen-2-propionaldehyd, para-Methylphenoxyacetaldehyd, 2-Methyl-3-phenyl-2-propen-1-al, 3,5,5-Trimethylhexanal, Hexahydro-8,8-dimethyl-2-naphthaldehyd, 3-Propylbicyclo[2.2.1]-hept-5-en-2-carbaldehyd, 9-Decenal, 3-Methyl-5-phenyl-1-pentanal, Methylnonylacetaldehyd, Hexanal sowie trans-2-Hexenal.

7. Wasch- oder Reinigungsmittel gemäß einem der Ansprüche 1 bis 6, **dadurch gekennzeichnet, dass** das Alken gemäß Formel (II), (IV) oder (VI) ausgewählt ist aus Riechstoffen mit allylischem Wasserstoffatom, insbesondere aus Limonen, α-Phellandren, β-Phellandren, α-Pinen, β-Pinen, Camphen, Caryophyllen, Longifolen, Ocimen, α-Terpinen, β-Terpinen, γ-Terpinen, α-Terpineol, δ-Terpineol, γ-Terpineol, β-Terpineol, α-Citronellol, β-Citronellol, α-Citronellal, β-Citronellal, Linalool, Dihydromyrcenol, Geraniol, Santalol, Hasmigone, Carvon, 2-Caren, 3-Caren, 4-Caren, Elemol und/oder Curcumen.

8. Wasch- oder Reinigungsmittel gemäß einem der Ansprüche 1 bis 7 **dadurch gekennzeichnet, dass** die Riechstoffzusammensetzung weitere Riechstoffe enthält.

9. Textilbeduftungsverfahren, **dadurch gekennzeichnet, dass** auf ein Textil ein Wasch- oder Reinigungsmittel gemäß einem der Ansprüche 1 bis 8 aufgebracht wird und die Oberfläche des Textils danach Temperaturen von ≥ 50 °C, vorzugsweise Temperaturen zwischen 60 und 250 °C ausgesetzt wird.

10. Aromagebungsverfahren bei der Nahrungszubereitung, **dadurch gekennzeichnet, dass** ein Nahrungsmittel, ein Nahrungsmittelvorprodukt oder ein Hilfsmittel der Nahrungsmittelzubereitung, welches jeweils eine Riechstoffzusammensetzung, enthaltend einen Allylether Riechstoffvorläufer der Formel (I) gemäß einem der Ansprüche 1 bis 8, erhitzt wird, so dass es Temperaturen > 50 °C ausgesetzt ist, insbesondere im Rahmen nahrungsmittelzubereitungsüblicher Vorgänge wie Kochen, Braten, Mikrowellengaren oder Backen.

## Claims

1. A washing or cleaning agent containing an odorant composition, in quantities between 0.0001 and 10wt.%, preferably between 0.001 and 5wt.%, more advantageously between 0.01 and 3wt.%, in particular between 0.1 and 2 wt.%, in each case relative to the entire agent, and containing an odorant precursor, which is an allyl ether of the formula (I),
R¹R²C=CR³-CR⁴R⁵-O-CHR⁶R⁷ (I),
wherein the residues R¹, R², R³, R⁴, R⁵, R⁶ and R⁷ mutually independently in each case denote H or a hydrocarbon residue which may be acyclic or cyclic, substituted or unsubstituted, branched or unbranched, as well as saturated or unsaturated, and wherein individual residues R¹, R², R³, R⁴, R⁵, R⁶, R⁷ may form a ring system with one another,
with the proviso that R¹, R², R³, R⁴ and R⁵ denote residues which, in a compound of the general formula (II)
R¹R²CH-CR³=CR⁴R⁵ (II),
give rise to an odorant,
and/or with the proviso that R⁶ and R⁷ denote residues which, in a compound of the general formula (III)
R⁶R⁷C=O (III),
give rise to an odorant.

2. A washing or cleaning agent according to claim 1, **characterised in that**, in the formula (I), the residues R² and R⁴ are hydrogen residues,
with the proviso that R¹, R³ and R⁵ denote residues which, in a compound of the general formula (IV)
R¹CH₂-CR³=CHR⁵ (IV),
give rise to an odorant,
and/or with the proviso that R⁶ and R⁷ denote residues which, in a compound of the general formula (III)
R⁶R⁷C=O (III),
give rise to an odorant.

3. A washing or cleaning agent according to claim 1, **characterised in that**, in the formula (I), the residues R², R⁴ and R⁶ are hydrogen residues,
with the proviso that R¹, R³ and R⁵ denote residues which, in a compound of the general formula (IV)
R¹CH₂-CR³=CHR⁵ (IV),
give rise to an odorant, and/or with the proviso that R⁷ denotes a residue which, in a compound of the general formula (V)
R⁷CHO (V),
gives rise to an odorant.

4. A washing or cleaning agent according to claim 1, **characterised in that**, in the formula (I), the residues R², R⁴, R⁵ and R⁶ are hydrogen residues, with the proviso that R¹ and R³ denote residues which, in a compound of the general formula (VI)
R¹CH₂-CR³=CH₂ (VI),
give rise to an odorant, and/or with the proviso that R⁷ denotes a residue which, in a compound of the general formula (V)
R⁷CHO (V),
gives rise to an odorant.

5. A washing or cleaning agent according to claim 1, **characterised in that**, in the formula (I), the residues R¹, R², R⁴ and R⁶ are hydrogen residues, with the proviso that R³ and R⁵ denote residues which, in a compound of the general formula (VI)
CH₃-CR³=CHR⁵ (VI)
give rise to an odorant,
and/or with the proviso that R⁷ denotes a residue which, in a compound of the general formula (V)
R⁷CHO (V),
gives rise to an odorant.

6. A washing or cleaning agent according to one of claims 1 to 5, **characterised in that** the compound of the general formula (III) or (V) is an odorant aldehyde, in particular selected from adoxal (2,6,10-trimethyl-9-undecenal), anisaldehyde (4-methoxybenzaldehyde), cymal (3-(4-isopropylphenyl)-2-methylpropanal), ethylvanillin, florhydral (3-(3-isopropylphenyl)butanal), helional (3-(3,4-methylenedioxyphenyl)-2-methylpropanal), heliotropin, hydroxycitronellal, lauraldehyde, lyral (3- and 4-(4-hydroxy-4-methylpentyl)-3-cyclohexene-1-carboxaldehyde), methylnonylacetaldehyde, lilial (3-(4-tert.-butylphenyl)-2-methylpropanal), phenylacetaldehyde, undecylenealdehyde, vanillin, 2,6,10-trimethyl-9-undecenal, 3-dodecen-1-al, alpha-n-amylcinnamaldehyde, melonal (2,6-dimethyl-5-heptenal), 2,4-dimethyl-3-cyclohexene-1-carboxaldehyde (triplal), benzaldehyde, 3-(4-tert.-butylphenyl)propanal, 2-methyl-3-(para-methoxyphenyl)propanal, 2-methyl-4-(2,6,6-trimethyl-2(1)-cyclohexen-1-yl)butanal, 3-phenyl-2-propenal, cis-/trans-3,7-dimethyl-2,6-octadien-1-al, 3,7-dimethyl-6-octen-1-al, [(3,7-dimethyl-6-octenyl)oxy]acetaldehyde, 4-isopropylbenzaldehyde, 1,2,3,4,5,6,7,8-octahydro-8,8-dimethyl-2-naphthaldehyde, 2,4-dimethyl-3-cyclohexene-1-carboxaldehyde, 2-methyl-3-(isopropylphenyl)propanal, 1-decanal, 2,6-dimethyl-5-heptenal, 4-(tricyclo[5.2.1.0(2,6)]-decylidene-8)-butanal, octahydro-4,7-methane-1H-indenecarboxaldehyde, 3-ethoxy-4-hydroxybenzaldehyde, para-ethyl-alpha,alpha-dimethylhydrocinnamaldehyde, alpha-methyl-3,4-(methylenedioxy)-hydrocinnamaldehyde, 3,4-methylenedioxybenzaldehyde, alpha-n-hexylcinnamaldehyde, m-cymene-7-carboxaldehyde, alpha-methylphenylacetaldehyde, 7-hydroxy-3,7-dimethyloctanal, undecenal, 2,4,6-trimethyl-3-cyclohexene-1-carboxaldehyde, 4-(3)(4-methyl-3-pentenyl)-3-cyclohexenecarboxaldehyde, 1-dodecanal, 2,4-dimethylcyclohexane-3-carboxaldehyde, 4-(4-hydroxy-4-methylpentyl)-3-cyclohexene-1-carboxaldehyde, 7-methoxy-3,7-dimethyloctan-1-al, 2-methylundecanal, 2-methyldecanal, 1-nonanal, 1-octanal, 2,6,10-trimethyl-5,9-undecadienal, 2-methyl-3-(4-tert.-butyl)propanal, dihydrocinnamaldehyde, 1-methyl-4-(4-methyl-3-pentenyl)-3-cyclohexene-1-carboxaldehyde, 5- or 6-methoxyhexahydro-4,7-methanindane-1- or -2-carboxaldehyde, 3,7-dimethyloctan-1-al, 1-undecanal, 10-undecen-1-al, 4-hydroxy-3-methoxybenzaldehyde, 1-methyl-3-(4-methylpentyl)-3-cyclohexenecarboxaldehyde, 7-hydroxy-3,7-dimethyloctanal, trans-4-decenal, 2,6-nonadienal, paratolylacetaldehyde, 4-methylphenylacetaldehyde, 2-methyl-4-(2,6,6-trimethyl-1-cyclohexen-1-yl)-2-butenal, ortho-methoxycinnamaldehyde, 3,5,6-trimethyl-3-cyclohexenecarboxaldehyde, 3,7-dimethyl-2-methylene-6-octenal, phenoxyacetaldehyde, 5,9-dimethyl-4,8-decadienal, peony aldehyde (6,10-dimethyl-3-oxa-5,9-undecadien-1-al), hexahydro-4,7-methanindane-1-carboxaldehyde, 2-methyloctanal, alpha-methyl-4-(1-methylethyl)benzeneacetaldehyde, 6,6-dimethyl-2-norpinene-2-propionaldehyde, para-methylphenoxyacetaldehyde, 2-methyl-3-phenyl-2-propen-1-al, 3,5,5-trimethylhexanal, hexahydro-8,8-dimethyl-2-naphthaldehyde, 3-propylbicyclo[2.2.1]hept-5-ene-2-carbaldehyde, 9-decenal, 3-methyl-5-phenyl-1-pentanal, methylnonylacetaldehyde, hexanal and trans-2-hexenal.

7. A washing or cleaning agent according to one of claims 1 to 6, **characterised in that** the alkene according to formula (II), (IV) or (VI) is selected from odorants with an allylic hydrogen atom, in particular from limonene, α-phellandrene, β-phellandrene, α-pinene, β-pinene, camphene, caryophyllene, longifolene, ocimene, α-terpinene, β-terpinene, γ-terpinene, α-terpineol, δ-terpineol, γ-terpineol, β-terpineol, α-citronellol, β-citronellol, α-citronellal, β-citronellal, linalool, dihydromyrcenol, geraniol, santalol, hasmigone, carvone, 2-carene, 3-carene, 4-carene, elemol and/or curcumene.

8. A washing or cleaning agent according to one of claims 1 to 7, **characterised in that** the composition contains further odorants.

9. A textile fragrancing method, **characterised in that** a washing or cleaning agent according to one of claims 1 to 8 is applied onto a textile and the surface of the textile is thereafter exposed to temperatures of ≥ 50°C, preferably temperatures of between 60 and 250°C.

10. An aromatisation method in foodstuff preparation, **characterised in that** a foodstuff, a foodstuff precursor or a foodstuff preparation auxiliary, which in each case contains an odorant composition containing an allyl ether odorant precursor of the formula (I) according to one of claims 1 to 8, is heated such that it is exposed to temperatures of > 50°C, in particular in the course of conventional food preparation procedures such as boiling, frying, microwaving or baking.

## Revendications

1. Agent de lavage ou de nettoyage, contenant une composition de matière odorante dans des quantités comprises entre 0,0001 et 10 % en poids, de préférence entre 0,001 et 5 % en poids, de manière encore plus avantageuse entre 0,01 et 3 % en poids, en particulier entre 0,1 et 2 % en poids, rapporté respectivement au produit total, contenant un précurseur de matière odorante, dans lequel il s'agit d'un éther allylique de la Formule (I),
R¹R²C=CR³-CR⁴R⁵-O-CHR⁶R⁷ (I),
dans laquelle les résidus R¹, R², R³, R⁴, R⁵, R⁶ et R⁷ représentent chacun indépendamment les uns des autres H ou un résidu hydrocarbure qui peut être acyclique ou cyclique, substitué ou non substitué, ramifié ou non ramifié, ainsi que saturé ou insaturé, et dans laquelle les résidus individuels R¹, R², R³, R⁴, R⁵, R⁶, R⁷ peuvent former ensemble un système cyclique,
à la condition que R¹, R², R³, R⁴ et R⁵ représentent des résidus qui résultent en une matière odorante dans un composé de la Formule générale (II)
R¹R²CH-CR³=CR⁴R⁵ (II),
et/ou à la condition que R⁶ et R⁷ représentent des résidus qui résultent en une matière odorante dans un composé de la Formule générale (III)
R⁶R⁷C=O (III).

2. Agent de lavage ou de nettoyage selon la revendication 1, **caractérisé en ce que** dans la Formule (I) les résidus R² ainsi que R⁴ sont des résidus hydrogène,
à la condition que R¹, R³ et R⁵ représentent des résidus qui résultent en une matière odorante dans un composé de la Formule générale (IV)
R¹CH₂-CR³=CHR⁵ (IV),
et/ou à la condition que R⁶ et R⁷ représentent des résidus qui résultent en une matière odorante dans un composé de la Formule générale (III)
R⁶R⁷C=O (III).

3. Agent de lavage ou de nettoyage selon la revendication 1, **caractérisé en ce que** dans la Formule (I) les résidus R², R⁴ ainsi que R⁶ sont des résidus hydrogène,
à la condition que R¹, R³ et R⁵ représentent des résidus qui résultent en une matière odorante dans un composé de la Formule générale (IV)
R¹CH₂-CR³=CHR⁵ (IV),
et/ou à la condition que R⁷ représente un résidu qui résulte en une matière odorante dans un composé de la Formule générale (V)
R⁷CHO (V).

4. Agent de lavage ou de nettoyage selon la revendication 1, **caractérisé en ce que** dans la Formule (I) les résidus R², R⁴, R⁵ ainsi que R⁶ sont des résidus hydrogène,
à la condition que R¹ et R³ représentent des résidus qui résultent en une matière odorante dans un composé de la Formule générale (VI)
R¹CH₂-CR³=CH₂ (VI),
et/ou à la condition que R⁷ représente un résidu qui résulte en une matière odorante dans un composé de la Formule générale (V)
R⁷CHO (V).

5. Agent de lavage ou de nettoyage selon la revendication 1, **caractérisé en ce que** dans la Formule (I) les résidus R¹, R², R⁴ ainsi que R⁶ sont des résidus hydrogène,
à la condition que R³ et R⁵ représentent des résidus qui résultent en une matière odorante dans un composé de la Formule générale (VI)
CH₃-CR³=CHR₅ (VI),
et/ou à la condition que R⁷ représente un résidu qui résulte en une matière odorante dans un composé de la Formule générale (V)
R⁷CHO (V).

6. Agent de lavage ou de nettoyage selon l'une des revendications 1 à 5, **caractérisé en ce que** le composé de la Formule générale (III) ou (V) est un aldéhyde odorant, sélectionné en particulier parmi l'adoxal (2,6,10-triméthyl-9-undécénal), l'anisaldéhyde (4-méthoxybenzaldéhyde), le cymal (3-(4-isopropylphényl)-2-méthylpropanal), l'éthylvanilline, le florhydral (3-(3-isopropylphényl)butanal), l'hélional (3-(3,4-méthylène dioxyphényl)-2-méthylpropanal), l'héliotropine, l'hydroxycitronellal, le lauraldéhyde, le lyral (3- et 4-(4-hydroxy-4-méthylpentyl)-3-cyclohexène-1-carboxaldéhyde), le méthylnonylacétaldéhyde, le lilial (3-(4-tert-butylphényl)-2-méthylpropanal), le phénylacétaldéhyde, l'undécylénaldéhyde, la vanilline, le 2,6,10-triméthyl-9-undécénal, le 3-dodécén-1-al, l'alpha-n-amylcinnamaldéhyde, le mélonal (2,6-diméthyl-5-hepténal), le 2,4-diméthyl-3-cyclohexène-1-carboxaldéhyde (triplal), le benzaldéhyde, le 3-(4-tert-butylphényl)propanal, le 2-méthyl-3-(para-méthoxyphényl)propanal, le 2-méthyl-4-(2,6,6-triméthyl-2(1)cyclohexén-1-yl)butanal, le 3-phényl-2-propénal, le cis-/trans-3,7-diméthyl-2,6-octadién-1-al, le 3,7-diméthyl-6-octén-1-al, le [(3,7-diméthyl-6-octényl)oxy]acétaldéhyde, le 4-isopropylbenzylaldéhyde, le 1,2,3,4,5,6,7,8-octahydro-8,8-diméthyl-2-naphtaldéhyde, le 2,4-diméthyl-3-cyclohexène-1-carboxaldéhyde, le 2-méthyl-3-(isopropylphényl)propanal, le 1-décanal, le 2,6-diméthyl-5-hepténal, le 4-(tricyclo[5.2.1.0(2,6)]-décylidène-8)-butanal, l'octahydro-4,7-méthane-1 H-indène carboxaldéhyde, le 3-éthoxy-4-hydroxybenzaldéhyde, le para-éthyl-alpha,alpha-diméthylhydrocinnamaldéhyde, l'alpha-méthyl-3,4-(méthylène dioxy)-hydrocinnamaldéhyde, le 3,4-méthylène dioxybenzaldéhyde, l'alpha-n-hexylcinnamaldéhyde, le m-cymène-7-carboxaldéhyde, l'alpha-méthylphénylacétaldéhyde, le 7-hydroxy-3,7-diméthyloctanal, l'undécénal, le 2,4,6-triméthyl-3-cyclohexène-1-carboxaldéhyde, le 4-(3)(4-méthyl-3-pentényl)-3-cyclohexène carboxaldéhyde, le 1-dodécanal, le 2,4-diméthylcyclohexène-3-carboxaldéhyde, le 4-(4-hydroxy-4-méthylpentyl)-3-cyclohexène-1-carboxaldéhyde, le 7-méthoxy-3,7-diméthyloctan-1-al, le 2-méthylundécanal, le 2-méthyldécanal, le 1-nonanal, le 1-octanal, le 2,6,10-triméthyl-5,9-undécadiénal, le 2-méthyl-3-(4-tert-butyl)propanal, le dihydrocinnamaldéhyde, le 1-méthyl-4-(4-méthyl-3-pentényl)-3-cyclohexène-1-carboxaldéhyde, le 5- ou 6-méthoxyhexahydro-4,7-méthane indan-1- ou -2-carboxaldéhyde, le 3,7-diméthyloctan-1-al, le 1-undécanal, le 10-undécén-1-al, le 4-hydroxy-3-méthoxybenzaldéhyde, le 1-méthyl-3-(4-méthylpentyl)-3-cyclohexène carboxaldéhyde, le 7-hydroxy-3,7-diméthyloctanal, le trans-4-décénal, le 2,6-nonadiénal, le para-tolylacétaldéhyde, le 4-méthylphénylacétaldéhyde, le 2-méthyl-4-(2,6,6-triméthyl-1-cyclohexén-1-yl)-2-buténal, l'ortho-méthoxycinnamaldéhyde, le 3,5,6-triméthyl-3-cyclohexène carboxaldéhyde, le 3,7-diméthyl-2-méthylène-6-octénal, le phénoxyacétaldéhyde, le 5,9-diméthyl-4,8-décadiénal, l'aldéhyde de pivoine (6,10-diméthyl-3-oxa-5,9-undécadién-1-al), l'hexahydro-4,7-méthane indane-1-carboxaldéhyde, le 2-méthyloctanal, l'alpha-méthyl-4-(1-méthyléthyl)benzèneacétaldéhyde, le 6,6-diméthyl-2-norpinène-2-propionaldéhyde, le para-méthylphénoxyacétaldéhyde, le 2-méthyl-3-phényl-2-propén-1-al, le 3,5,5-triméthylhexanal, l'hexahydro-8,8-diméthyl-2-naphtaldéhyde, le 3-propylbicyclo[2.2.1]hept-5-ène-2-carbaldéhyde, le 9-décénal, le 3-méthyl-5-phényl-1-pentanal, le méthylnonylacétaldéhyde, l'hexanal ainsi que le trans-2-hexénal.

7. Agent de lavage ou de nettoyage selon l'une des revendications 1 à 6, **caractérisé en ce que** l'alcène selon la Formule (II), (IV) ou (VI) est sélectionné parmi des matières odorantes avec un atome d'hydrogène allylique, en particulier parmi le limonène, l'α-phellandrène, le ß-phellandrène, l'α-pinène, le ß-pinène, le camphène, le caryophyllène, le longifolène, l'ocimène, l'α-terpinène, le ß-terpinène, le γ-terpinène, l'α-terpinéol, le δ-terpinéol, le γ-terpinéol, le ß-terpinéol, l'α-citronellol, le ß-citronellol, l'α-citronellal, le ß-citronellal, le linalool, le dihydromyrcénol, le géraniol, le santalol, l'hasmigone, la carvone, le 2-carène, le 3-carène, le 4-carène, l'élémol et/ou le curcumène.

8. Agent de lavage ou de nettoyage selon l'une des revendications 1 à 7, **caractérisé en ce que** la composition de matière odorante contient d'autres matières odorantes.

9. Procédé de parfumage de textile, **caractérisé en ce qu'**un agent de lavage ou de nettoyage selon l'une des revendications 1 à 8 est appliqué sur un textile et la surface du textile est ensuite soumise à des températures de ≥ 50 °C, de préférence des températures comprises entre 60 et 250 °C.

10. Procédé d'aromatisation dans la préparation de nourriture, **caractérisé en ce qu'**un aliment, un produit alimentaire semi-fini ou un adjuvant de la préparation alimentaire, lequel contient respectivement une composition de matière odorante, contenant un précurseur de matière odorante éther allylique de la Formule (I) selon l'une des revendications 1 à 8, est chauffé de sorte qu'il soit soumis à des températures > 50 °C, en particulier dans le cadre de processus habituels d'une préparation d'aliments comme la cuisson bouillie, le rôtissage, la cuisson au micro-ondes ou la cuisson au four.
